# EUROPEAN PATENT APPLICATION

(11) **EP 4 332 239 A1**
(43) Date of publication of application: **06.03.2024**
(21) Application number: 22192866.6
(22) Date of filing: 30.08.2022
(51) Int. Cl.: C12Q 1/6886

(54) **MIR-BASED ASSAY FOR GASTRO-ENTERO-PANCREATIC NEUROENDOCRINE TUMOR DIAGNOSIS AND PROGNOSIS**

(71) Applicant: Istituto Romagnolo per lo Studio dei Tumori "Dino Amadori" - IRST S.r.l., 47014 Meldola (FC) (IT)
(72) Inventor: Paganelli, Giovanni, 47014 Meldola (FC) (IT); Mazza, Massimiliano, 47014 Meldola (FC) (IT); Bocchini, Martine, 47014 Meldola (FC) (IT); Nicolini, Fabio, 47014 Meldola (FC) (IT)
(74) Representative: Ghirardi, Valeria

(57) **Abstract**

The present invention refers to the diagnosis, prognosis and treatment of gastro-entero-pancreatic neuroendocrine tumors (GEP-NET). In particular, it refers to an *in vitro* method for diagnosing a gastro-entero-pancreatic neuroendocrine tumor (GEP-NET) in a subject and/or determining aggressiveness of a GEP-NET in a subject diagnosed with GEP-NET and/or predicting overall survival of a subject with a GEP-NET wherein the method comprises the step of determining the expression level of one or more miRNAs in a biological sample comprising blood previously obtained from said subject, wherein the miRNAs are selected from: hsa-miR-5096, hsa-let-7i-3p and hsa-miR-4311.

## Description

### FIELD OF THE INVENTION

The present invention refers to the field of diagnosis, prognosis and treatment of gastro-entero-pancreatic neuroendocrine tumors (GEP-NET).

In particular, it refers to the use of miRNAs for the diagnosis, prognosis and treatment of pancreatic NETs.

### BACKGROUND

Gastro-entero-pancreatic neuroendocrine tumors (GEP-NETs) are rare and heterogeneous malignancies arising from the diffuse neuroendocrine system. NET disease exhibits variable aggressiveness depending on the site of origin, grade, stage, and functionality¹. Pancreatic NETs (P-NETs) represent less than 5% of all pancreatic cancers, although incidence and prevalence are rising². P-NETs include tumors with a wide spectrum of clinical behaviors, thus often indolent and diagnosed in advanced stage³

Nowadays, P-NETs can be diagnosed earlier and updated therapeutic algorithms and guidelines have been proposed ^{4,5,6,7,8,9}. Recent important updates in the 5th edition (2019) of the World Health Organization (WHO) identify a novel G3 P-NET molecular subset, which includes well differentiated (WD) tumors with high proliferative index (> 20%) ^{10,11}.

Despite novel classification helps the stratification of patients, improving prognosis and response to treatment¹², substantial differences in clinical behavior still remain, making personalized treatment challenging for advanced P-NET^{6,13.}

Molecular imaging with positron emission tomography/computed tomography (PET/CT) holds a crucial role in P-NETs management. Updated European Neuroendocrine Society (ENETS) consensus on Radiological, Nuclear Medicine & Hybrid Imaging recommended ⁶⁸Ga-DOTA-somatostatin analog-PET/CT for tumor staging, preoperative imaging, and re-staging. PET with ⁶⁸Ga-DOTA peptides reveals tissues over-expressing somatostatin receptors (SSTRs). SSTR2 is expressed in 50% to 100% of P-NETS¹⁴.

Although the sensitivity and specificity of SSTR2-specific ⁶⁸Ga-DOTATATE PET/CT imaging has been proven, its clinical utility is hampered by heterogeneous SSTR2 expression among patients. Patients with low SSTR2 expression are not eligible for SSTR2-based imaging or therapies ^{15,16} In addition, poorly differentiated NET, can no longer concentrate somatostatin analogs, likely because of SSTRs expression loss. Therefore, the capability to restore high levels of functional SSTR2 in NET patients would improve imaging, the assessment of tumor burden and the efficacy of SSTR2-targeting therapies.

In this context, high SSTR expression can be considered a crude predictor of response to treatment, making patients eligible for Peptide Receptor Radionuclide Therapy (PRRT), notwithstanding PET/CT scan with ⁶⁸Ga-DOTA peptides alone does not represent a prognostic parameter in terms of progression-free survival (PFS)¹⁷.

On the other hand, ¹⁸F-FDG-PET/CT functional imaging is recommended for G3 and high G2 NETs, which generally display higher glucose metabolism and aggressiveness. This kind of lesion lacks SSTR2 overexpression and may fail to be detected by ⁶⁸Ga-DOTA-somatostatin analog-PET/CT¹⁷. Besides the diagnostic purpose of ¹⁸F-FDG-PET/CT in revealing lesions with higher metabolism, its prognostic value for NETs, especially for P-NETs has been established ^{18,19}. ¹⁸F-FDG positive lesions at PET/CT scan are associated with worse prognosis, aggressive tumor behavior and resistance to PRRT²⁰. From this perspective, high glucose consumption seems associated with lower radiosensitivity. This could be related to the triggering of proliferative pathways and/or molecular alterations that could drive the tumor resistance to PRRT or relapse. Although PRRT extends PFS, about 15-30% of patients progress during treatment or six months to one year after PRRT²¹⁻²³. Future optimization of PRRT will depend on improved pretreatment stratification and patient selection based on solid indicators of response²⁴.

In view of the above, it is evident that there is an urgent unmet clinical need for novel prognostic and predictive biomarkers which can supplement grade, stage, and imaging to address more tailored treatments for P-NET patients, especially for the advanced stage ones^{3,25}.

Blood biomarkers are simple to assess, minimally invasive, can be used for real-time and longitudinal measurements and are reproducible, quantitative and objective. Moreover, liquid markers overcome limitations of tissue specific information, providing a real-time snapshot of the whole disease. MiRNAs are useful markers to diagnose and monitor disease progression since they are stable, mostly encapsulated in exosomes and retrievable from body fluids.

The National Institute of Health (NIH) has established three broad categories of biomarkers based on clinical utility²⁶. This includes type 0 markers, 'indicators of the natural history of disease', which correlate with diagnosis, prognosis, and outcome: both directly and indirectly. Type I 'captures the effects of an intervention according to the specific mechanism of action of the drug' and reflects the general efficacy of treatment through a defined mechanism of action. Type II markers are defined as surrogates for clinical endpoints, reflecting patient health, functionality, or survival.

A commercially available assay is the NETest that, up to now, represents the gold standard for NETs diagnosis. The NETest assay relies on the simultaneous detection of dozens of mRNA transcripts (n.53) in blood which can provide predictive information, such as clinical outcome, by means of highly complicated algorithms. Its principal clinical utility is to monitor disease progression and help prognostication²⁷. More recently, the NETest was combined with grade and used as a PRRT predictive quotient (PPQ) to predict efficacy of PRRT treatment in NETs^{28,29}. However, up to now, NETest outcome data are managed, analysed and interpreted by a single laboratory only worldwide.

No studies reported the relation of NETest with 18FDG-PET/CT, which represent a prognostic biomarker, especially for pancreatic disease. There are no commercial kits able to identify the 18FDG-PET/CT status in PanNET patients. Despite NETest has been reported to display high overall diagnostic accuracy in determining the either presence (rule-in) or absence (rule-out) of a disease (candidate "Type 0 " biomarker), few information is provided on its role in distinguishing NET from different sites of origin. It is well established that for certain NET diseases the primary tumor site of origin is unknown or difficult to determine, in addition PanNETs and SINETs display different prognosis and may benefits of differentiated treatments.

It has now been found a novel, liquid miRNA signature with diagnostic, prognostic and predictive features for the management of gastro-entero-pancreatic neuroendocrine tumors, in particular of pancreatic neuroendocrine tumors. In particular, said miRNA signature is a new accurate biomarker for functional imaging, metabolism and survival.

### SUMMARY OF THE INVENTION

It has now been found that miRNAs hsa-miR-5096, hsa-let-7i-3p and hsa-miR-4311 have a robust correlation with 18F-FDG-PET status in P-NET patients (p <0·005). In particular hsa-miR-5096 was found to: i) predict 6-month Progression Free Survival (6mo-PFS), and 12-month overall survival (12mo-OS) for patients treated with PRRT (p<0·001 and p<0 05, respectively); ii) identify 18F-FDG-PET positive P-NET patients with worse prognosis after PRRT (p<0·005); iii) anti-correlate with SSTR2 expression on P-NET tissue and with the maximum standardized uptake (SUVmax) of 68Ga-PET (p<0·05) and with SSTRs expression; iv) decrease SSTR2 levels when ectopically expressed in P-NET cells (p<0·01).

Said miRNAs can therefore be advantageously used as biomarkers in a variety of diagnostic and prognostic applications regarding GEP-NET tumors which have been found to correlate to the expression levels of such miRNAs in the blood of a subject.

Advantageously, the miRNAs-signature of the invention can be easily assessed in potentially every hospital/laboratory equipped with basic instruments for molecular diagnosis (e.g. RT-PCR) that measure their relative abundance or use a more standardized solution, such as kit/assay/tool. The identification of said miRNAs as therapeutic targets also constitutes an independent track for products or for the design of novel therapeutic strategies. Therapies based on hsa-miR-5096-5p inhibition can indeed sensitize the tumor to SSTR-2 targeted therapies (i.e. PRRT).

It is an object of the invention the use of hsa-miR-5096, hsa-let-7i-3p and/or hsa-miR-4311 as biomarkers for diagnosing a gastro-entero-pancreatic neuroendocrine tumor (GEP-NET) in a subject and/or determining aggressiveness of a GEP-NET in a subject diagnosed with GEP-NET and/or predicting overall survival of a subject with a GEP-NET and/or predicting response to therapy in a subject with a GEP-NET.

In particular, it is an object of the invention the use of hsa-miR-5096, hsa-let-7i-3p and/or hsa-miR-4311 as biomarkers for:
- determining the presence of a gastro-entero-pancreatic neuroendocrine tumor (GEP-NET), in particular of a pancreatic neuroendocrine tumor (P-NET) or of a Small Intestine neuroendocrine tumor (SI-NET);
- diagnosing an ileal primary site of origin in a subject affected by Small Intestine NET (SI-NET);
- determining the presence of 18F-FDG-PET positive lesions in a subject affected by a pancreatic neuroendocrine tumor (P-NET);
- predicting 6 month progression-free survival (PFS) in a subject affected by a pancreatic neuroendocrine tumor (P-NET) who is undergoing a treatment with Peptide Radionuclide Receptor Therapy (PRRT);
- predicting 12 month overall survival (OS) in a subject affected by a pancreatic neuroendocrine tumor (P-NET) who is undergoing a treatment with Peptide Radionuclide Receptor Therapy (PRRT);
- predicting 6 month progression-free survival (PFS) in a subject affected by a pancreatic neuroendocrine tumor (P-NET) who is undergoing a treatment with Peptide Radionuclide Receptor Therapy (PRRT) and is positive to ¹⁸F-FDG-PET;
- predicting the presence of highly proliferating P-NET lesions in a subject affected by a pancreatic neuroendocrine tumor (P-NET);
- grading a pancreatic neuroendocrine tumor (P-NET);
- predicting tumor burden in a subject affected by a pancreatic neuroendocrine tumor (P-NET);
- predicting response to Peptide Radionuclide Receptor Therapy (PRRT) in a subject affected by a pancreatic neuroendocrine tumor (P-NET).

It is an object of the invention an *in vitro* method of diagnosing a gastro-entero-pancreatic neuroendocrine tumor (GEP-NET) in a subject and/or determining aggressiveness of a GEP-NET in a subject diagnosed with GEP-NET and/or predicting overall survival of a subject with a GEP-NET wherein the method comprises the step of determining the expression level of one or more miRNAs in a biological sample comprising blood previously obtained from said subject, wherein the miRNAs are selected from: hsa-miR-5096, hsa-let-7i-3p and hsa-miR-4311.

In particular, it is an object of the invention an *in vitro* method for:
- determining the presence of a gastro-entero-pancreatic neuroendocrine tumor (GEP-NET) in a subject, in particular of a pancreatic neuroendocrine tumor (P-NET) or a Small Intestine neuroendocrine tumor (SINET);
- diagnosing an ileal primary site of origin in a subject affected by Small Intestine NET (SINET);
- determining the presence of 18F-FDG-PET positive lesions in a subject affected by a pancreatic neuroendocrine tumor (P-NET);
- predicting 6 month progression-free survival (PFS) in a subject affected by a pancreatic neuroendocrine tumor (P-NET), in particular who is undergoing a treatment with Peptide Radionuclide Receptor Therapy (PRRT);
- predicting 12 month overall survival (OS) in a subject affected by a pancreatic neuroendocrine tumor (P-NET), in particular who is undergoing a treatment with Peptide Radionuclide Receptor Therapy (PRRT);
- predicting 6 month progression-free survival (PFS) in a subject affected by a pancreatic neuroendocrine tumor (P-NET) who is undergoing a treatment with Peptide Radionuclide Receptor Therapy (PRRT) and is positive to ¹⁸F-FDG-PET;
- predicting the presence of highly proliferating P-NET lesions in a subject affected by a pancreatic neuroendocrine tumor (P-NET);
- grading a pancreatic neuroendocrine tumor (P-NET);
- predicting tumor burden in a subject affected by a pancreatic neuroendocrine tumor (P-NET); and/or
- predicting response to Peptide Radionuclide Receptor Therapy (PRRT) in a subject affected by a pancreatic neuroendocrine tumor (P-NET),
comprising the step of determining the expression level of hsa-miR-5096 and/or hsa-let-7i-3p and/or hsa-miR-4311 and/or combinations thereof in a biological sample comprising blood obtained from said subject.

Preferably the expression level of the one or more miRNAs is normalized in relation to the expression levels of one or more reference molecule, such as a miRNA, a RNA, a DNA or other biomolecule, in particular a molecule which does not change its amount in a subject with a GEP-NET with respect to a healthy subject.

Preferably, the method of the invention further comprises a step of determining a diagnostic or prognostic response by means of a comparison between the one or more miRNAs and a reference molecule. Said reference molecule can be any molecule which does not change its amount in a subject with a GEP-NET with respect to a healthy subject, for example it can be a miRNA, RNA, DNA or other biomolecule. Said response is preferably diagnosing a gastro-entero-pancreatic neuroendocrine tumor (GEP-NET) and/or determining aggressiveness of a GEP-NET and/or predicting overall survival of a subject with a GEP-NET.

In an embodiment, a cut-off is determined based on the chosen reference molecule and the response is determined if the normalized expression levels of the one or more miRNAs are below or above said cut-off.

Particular embodiments of the methods of the invention will be described below.

In all such methods, preferably the expression level of the one or more miRNAs is normalized in relation to the expression levels of one or more reference molecule, such as a miRNA, a RNA, a DNA or other biomolecule, in particular a molecule which does not change its amount in a subject with a GEP-NET with respect to a healthy subject. In a preferred embodiment, said reference molecule is a miRNA, more preferably it is hsa-miR-30d.

In all such methods, preferably, the method further comprises the step of determining a response by means of a comparison between the one or more miRNAs and a reference molecule. In some embodiments, a cut-off is determined based on the chosen reference molecule and the response is determined if the normalized expression levels of the one or more miRNAs are below or above said cut-off. The skilled person is able to choose a suitable reference molecule and consequently determine the relevant cut-off according to common general knowledge in the field.

A gastroenteropancreatic neuroendocrine tumor (GEP-NET) is a rare type of tumor that can grow in the pancreas or other areas of the gut, such as the stomach, small intestine, rectum, colon, or appendix. Preferably, the GEP-NET is selected from pancreatic neuroendocrine tumor (P-NET), small-intestine neuroendocrine tumor (SI-NETS), intestine neuroendocrine tumor, stomach neuroendocrine tumor.

In an embodiment, it is an object of the invention an *in vitro* method for diagnosing a gastro-entero-pancreatic neuroendocrine tumor (GEP-NET) in a subject in need thereof comprising the step of determining the expression of miRNAs hsa-miR-5096, hsa-let-7i-3p and/or hsa-miR-4311 in a biological sample comprising blood previously obtained from said subject. In particular the method can provide differential diagnosis of NETs of pancreatic origin (P-NETS) or of small-intestine origin (SI-NETS).

In particular, it is an object of the invention an in vitro method to diagnose the presence of a Small Intestine neuroendocrine tumor (SI-NET) in a subject comprising the step of determining the expression level of hsa-miR-5096 and/or hsa-let-7i-3p in a biological sample comprising blood previously obtained from said subject.

In a preferred embodiment, it is an object of the invention an in vitro method to diagnose the presence of a Small Intestine neuroendocrine tumor (SI-NET) in a subject comprising the following steps:
a) determining the expression level of hsa-miR-5096 and/or hsa-let-7i-3p in a blood sample from the subject;
b) normalizing the expression level of each miRNA determined in step a) to the expression level of a reference miRNA, RNA, DNA or other biomolecule which does not change its amount in patients bearing a SI-NET with respect to a healthy subject;
c) determining the presence of Small Intestine NET (SI-NET) when the normalized expression levels of hsa-miR-5096 or of a combination of hsa-miR-5096 and hsa-let-7i-3p are equal to or greater than a predetermined cut off value.

It is a further object of the invention an in vitro method for diagnosing an ileal primary site of origin in a subject affected by Small Intestine NET (SI-NET) comprising the step of determining the expression level of hsa-miR-5096 and/or hsa-let-7i-3p in a biological sample comprising blood previously obtained from said subject.

It is a further object of the invention a method for diagnosing an ileal primary site of origin in a subject affected by Small Intestine NET (SI-NET) comprising the following steps:
a) determining the expression level of hsa-miR-5096 and/or hsa-let-7i-3p in a blood sample from the subject;
b) normalizing the expression level of each miRNA determined in step a) to the expression level of a reference miRNA, RNA, DNA or other biomolecule which does not change its amount in patients bearing a SI-NET with respect to a healthy subject;
c) determining the presence of an ileal primary site of origin when the normalized expression level of each of the miRNAs or of a combination thereof is equal to or greater than a predetermined cut off value.

A further object of the invention is a method to distinguish 18F-FDG-PET positive from 18F-FDG-PET negative gastro-entero-pancreatic neuroendocrine tumor (GEP-NET) patients, in particular P-NET patients, wherein 18F-FDG-PET negative tumors are characterized by less aggressive disease and better prognosis after Peptide Radionuclide Receptor Therapy (PRRT).

It is therefore an object of the invention an in vitro method to determine the presence of 18F-FDG-PET positive lesions in a subject affected by a gastro-entero-pancreatic neuroendocrine tumor (GEP-NET) , preferably a pancreatic neuroendocrine tumor (P-NET), comprising the step of determining the expression level of at least one of the following miRNAs: hsa-miR-5096, hsa-let-7i-3p and hsa-miR-4311 in a biological sample comprising blood previously obtained from said subject, wherein the presence of 18F-FDG-PET positive lesions is an indication of an aggressive disease.

In a preferred embodiment, it is determined the expression level of both hsa-let-7i-3p and hsa-miR-5096.

In particular, it is an object of the invention a method to determine the presence of 18F-FDG-PET positive lesions in a subject affected by a pancreatic neuroendocrine tumor (P-NET) comprising the following steps:
a) determining the expression level of at least one of the following miRNAs: hsa-miR-5096, hsa-let-7i-3p and hsa-miR-4311 in a blood sample from the subject;
b) normalizing the expression level of each miRNA determined in step a) to the expression level of a reference miRNA, RNA, DNA or other biomolecule which does not change its amount in patients bearing a P-NET with respect to a healthy subject;
c) determining the presence of at least one 18F-FDG-PET positive lesion when the normalized expression level of each of the miRNAs or of a combination thereof is equal to or greater than a predetermined cut off value.

In a preferred embodiment, said reference miRNA is hsa-miR-30d and said predetermined cut off value is:
0.85 for hsa-miR-4311;
70 for hsa-miR-5096;
0.72 for hsa-miR-let7i-3p;
0.65 for the combination of hsa-miR-4311 and hsa-let-7i-3p;
33.55 for the combination of hsa-mir-5096 and hsa-let-7i-3p;
14.9 for the combination of hsa-miR-4311 and hsa-mir-5096;
29.6 for the combination of hsa-miR-4311, hsa-mir-5096 and hsa-let-7i-3p,
wherein each cut-off value can vary of +/- 10%.

In a preferred embodiment, in step a) it is determined the expression level of both hsa-let-7i-3p and hsa-miR-5096.

It is a further object of the invention an in vitro method to predict 6 month progression-free survival (PFS) in a subject affected by a gastro-entero-pancreatic neuroendocrine tumor (GEP-NET), preferably a pancreatic neuroendocrine tumor (P-NET), comprising the step of determining the expression level of at least one of the following miRNAs: hsa-miR-5096, hsa-let-7i-3p and hsa-miR-4311 in a biological sample comprising blood previously obtained from said subject. Preferably, the expression level of hsa-miR-5096 is determined.

In particular it is an object of the invention a method to predict 6 month progression-free survival (PFS) in a subject affected by a pancreatic neuroendocrine tumor (P-NET) comprising the following steps:
a) determining the expression level of at least one of the following miRNAs: hsa-miR-5096, hsa-let-7i-3p and hsa-miR-4311 in a blood sample from said subject;
b) normalizing the expression level of each miRNA determined in step a) to the expression level of a reference miRNA, RNA, DNA or other biomolecule which does not change its amount in patients bearing a P-NET with respect to a healthy subject;
c) predicting a PFS of less than 6 months when the normalized expression level of each of the miRNAs or of a combination thereof is equal to or greater than a predetermined cut off value.

In a preferred embodiment, said reference miRNA is hsa-miR-30d and said predetermined cut off value is:
70 for hsa-miR-5096;
123.3 for the combination of hsa-mir-5096 and hsa-let-7i-3p;
142.9 for the combination of hsa-miR-4311 and hsa-mir-5096;
108.3 for the combination of hsa-miR-4311, hsa-mir-5096 and hsa-let-7i-3p,
wherein each cut-off value can vary by +/- 10%.

In a preferred embodiment, in step a) it is determined the expression level of hsa-miR-5096.

Preferably, the subject is undergoing a therapy treatment targeting somatostatin receptors, more preferably a treatment with Peptide Radionuclide Receptor Therapy (PRRT).

In a preferred embodiment, the subject affected by a pancreatic neuroendocrine tumor (P-NET) is undergoing a treatment with Peptide Radionuclide Receptor Therapy (PRRT) and is positive to ¹⁸F-FDG-PET.

It is a further object of the invention an in vitro method to predict 12 month overall survival (OS) in a subject affected by a gastro-entero-pancreatic neuroendocrine tumor (GEP-NET), preferably a pancreatic neuroendocrine tumor (P-NET), comprising the step of determining the expression level of hsa-miR-5096 in a biological sample comprising blood previously obtained from said subject.

In particular, it is an object of the invention a method to predict 12 month overall survival (OS) in a subject affected by a pancreatic neuroendocrine tumor (P-NET) comprising the following steps:
a) determining the expression level of hsa-miR-5096 in a blood sample from said subject;
b) normalizing the expression level of hsa-miR-5096 to a reference miRNA, RNA, DNA or other biomolecule which does not change its amount in patients bearing a P-NET with respect to a healthy subject;
c) predicting an overall survival of less than 12 months when the normalized expression level of hsa-miR-5096 is equal to or greater than a predetermined cut off value.

In a preferred embodiment, said reference miRNA is hsa-miR-30d and said predetermined cut off value is 70+/-10%.

Preferably, the subject is undergoing a therapy treatment targeting somatostatin receptors, more preferably a treatment with Peptide Radionuclide Receptor Therapy (PRRT).

It is a further object of the invention an in vitro method for predicting the presence of one or more highly proliferating tumor lesions in a gastro-entero-pancreatic neuroendocrine tumor (GEP-NET) comprising the step of determining the expression level of at least one of the following miRNAs: hsa-let-7i-3p and hsa-miR-4311, preferably miR-4311, in a biological sample comprising blood obtained from said subject. Preferably, the tumor is a pancreatic neuroendocrine tumor (P-NET).

In particular, it is a further object of the invention a method for predicting the presence of one or more highly proliferating tumor lesions in a subject affected by a pancreatic neuroendocrine tumor (P-NET) comprising the following steps:
a) determining the expression level of at least one of the following miRNAs: hsa-let-7i-3p and hsa-miR-4311 in a blood sample from said subject;
b) normalizing the expression level of each miRNA determined in step a) to the expression level of a reference miRNA, RNA, DNA or other biomolecule which does not change its amount in patients bearing a P-NET with respect to a healthy subject;
c) determining the presence of a highly proliferating P-NET lesion when the normalized expression level of each of the miRNAs or of a combination thereof is equal to or greater than a predetermined cut off value.

In a preferred embodiment, said reference miRNA is hsa-miR-30d and the predetermined cut off value is:
1.44 for hsa-miR-4311;
1.31 for hsa-let-7i-3p;
0.98 for the combination of hsa-miR-4311 and hsa-let-7i-3p;
wherein each cut-off value can vary of +/- 10%.

In a preferred embodiment, in step a) the expression level of hsa-miR-4311 is determined.

It is a further object of the invention an in vitro method for grading a gastro-entero-pancreatic neuroendocrine tumor (GEP-NET), in particular a pancreatic neuroendocrine tumor (P-NET), in the highest grade G3 comprising the step of determining the expression level of the combination of the following miRNAs: hsa-miR-5096, hsa-let-7i-3p and hsa-miR-4311 in a biological sample comprising blood previously obtained from a subject affected by a pancreatic neuroendocrine tumor.

In particular, it is an object of the invention a method for grading a pancreatic neuroendocrine tumor (P-NET) in the highest grade G3 comprising the following steps:
a) determining the expression level of the combination of the following miRNAs: hsa-miR-5096, hsa-let-7i-3p and hsa-miR-4311 in a blood sample from a subject affected by pancreatic neuroendocrine tumor;
b) normalizing the expression level of each miRNA determined in step a) to the expression level of a reference miRNA, RNA, DNA or other biomolecule which does not change its amount in patients bearing a P-NET with respect to a healthy subject;
c) determining the presence of a G3 P-NET when the normalized expression level of the combination of miRNAs of step a) is equal to or greater than a predetermined cut off value.

In a preferred embodiment, said reference miRNA is hsa-miR-30d and the cut-off value is 57.1 +/-10%.

It is a further object of the invention an in vitro method for determining tumor burden in a subject affected by a gastro-entero-pancreatic neuroendocrine tumor (GEP-NET), preferably a pancreatic neuroendocrine tumor (P-NET), comprising the step of determining the expression level of at least one of the following miRNAs: hsa-let-7i-3p and hsa-miR-4311 in a biological sample comprising blood obtained from said subject.

In particular, it is an object of the invention a method for determining tumor burden in a subject affected by a pancreatic neuroendocrine tumor (P-NET) comprising the following steps:
a) determining the expression level of at least one of the following miRNAs: hsa-let-7i-3p and hsa-miR-4311 in a blood sample from said subject;
b) normalizing the expression level of each miRNA determined in step a) to the expression level of a reference miRNA, RNA, DNA or other biomolecule which does not change its amount in patients bearing a P-NET with respect to a healthy subject;
c) determining the presence of a high tumor burden when the normalized expression level of the combination of miRNAs of step a) is equal to or greater than a predetermined cut off value.

It is a further object of the invention an in vitro method to predict response to therapy in a subject affected by a gastro-entero-pancreatic neuroendocrine tumor (GEP-NET), in particular a pancreatic neuroendocrine tumor (P-NET), comprising the step of determining the expression level of hsa-miR-5096, in a biological sample comprising blood previously obtained from said subject.

It is a further object of the invention a method to predict response to Peptide Radionuclide Receptor Therapy (PRRT) in a subject affected by a pancreatic neuroendocrine tumor (P-NET) comprising the following step:
a) determining the expression level of hsa-miR-5096 in a blood sample from said subject;
b) normalizing the expression level of each miRNA determined in step a) to the expression level of a reference miRNA, RNA, DNA or other biomolecule which does not change its amount in patients bearing a P-NET with respect to a healthy subject;
c) predicting a positive response to Peptide Radionuclide Receptor Therapy (PRRT) when the normalized expression level of hsa-miR-5096 is lower than a predetermined cut off value.

In a preferred embodiment, said reference miRNA is hsa-miR-30d and the cut off value is 70 +/-10%.

It is a further object of the invention an inhibitor of hsa-miR-5096 for use for the treatment of human cancers, preferably gastro-entero-pancreatic neuroendocrine tumors (GEP-NETs) and in particular pancreatic neuroendocrine tumors (P-NET).

It is also an object of the invention, a kit or a device for carrying out any of the methods above disclosed.

### DETAILED DISCLOSURE OF THE INVENTION

### Figures

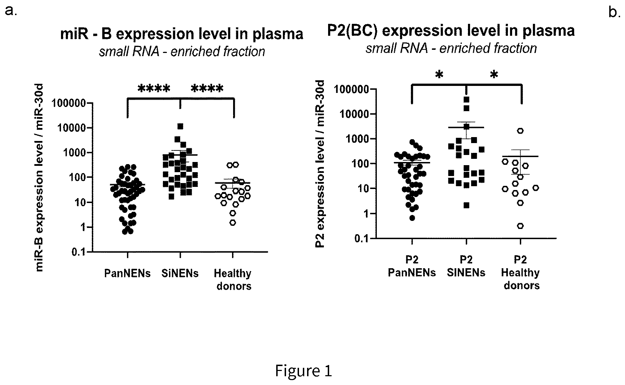

**Figure 1** **Circulating exosomal hsa-miR-5096 overexpression can identify P-NETs from SI-NET patients** (a) miR-5096 is significantly overexpressed in P-NETs when compared to SI-NET patients and Healthy Donors; (b) P2 (hsa-mir-5096^{∗}hsa-let-7i-3p) is significantly overexpressed in P-NETs when compared to SI-NET patients and Healthy Donors.

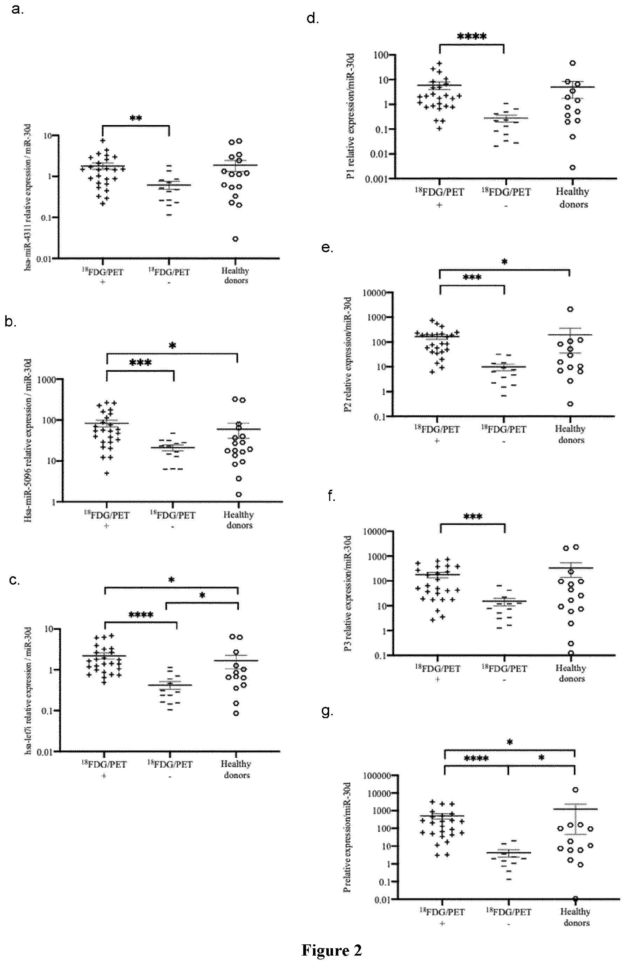

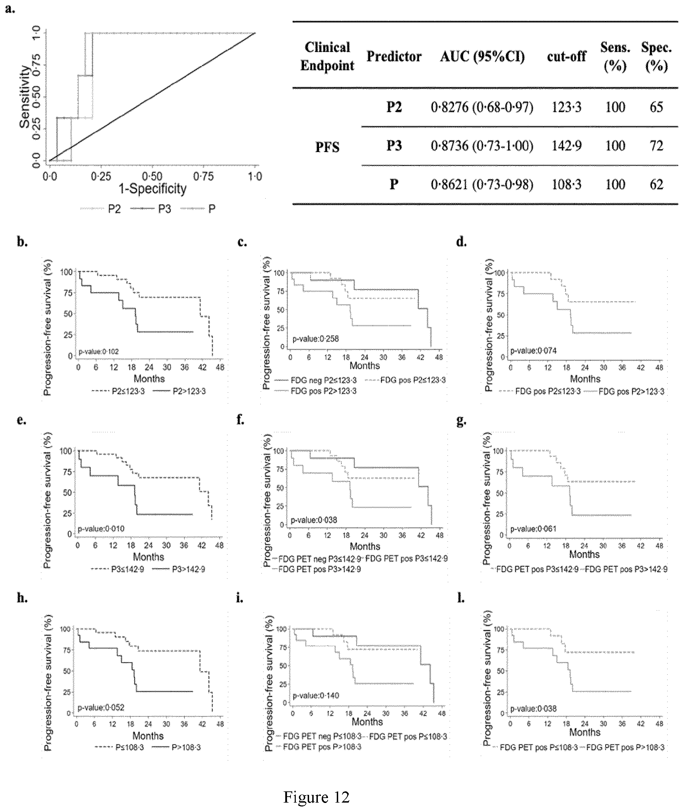

**Figure 2** **NGS analysis of circulating exosomal miRNAs in GEP-NETs revealed a metabolic signature in P-NET patients** (a-g) Deregulated miRNAs and predictor between ¹⁸F-FDG/PET positive and negative P-NETs: (a) hsa-miR-4311 (b) hsa-miR-5096 and (c) hsa-let-7i-3p and combined predictors (d) P1 (hsa-miR-4311^{∗} hsa-let-7i-3p), (e) P2 (hsa-mir-5096^{∗}hsa-let-7i-3p), (f) P3 (hsa-miR-4311^{∗} hsa-mir-5096), (g) P (hsa-miR-4311^{∗}hsa-mir-5096^{∗}hsa-let-7i-3p). Hsa-miR-30d was selected from NGS profiling as an endogenous control. Results are presented as mean ± SD (^{∗}P<0·05; ^{∗∗∗∗}P<0·00005).

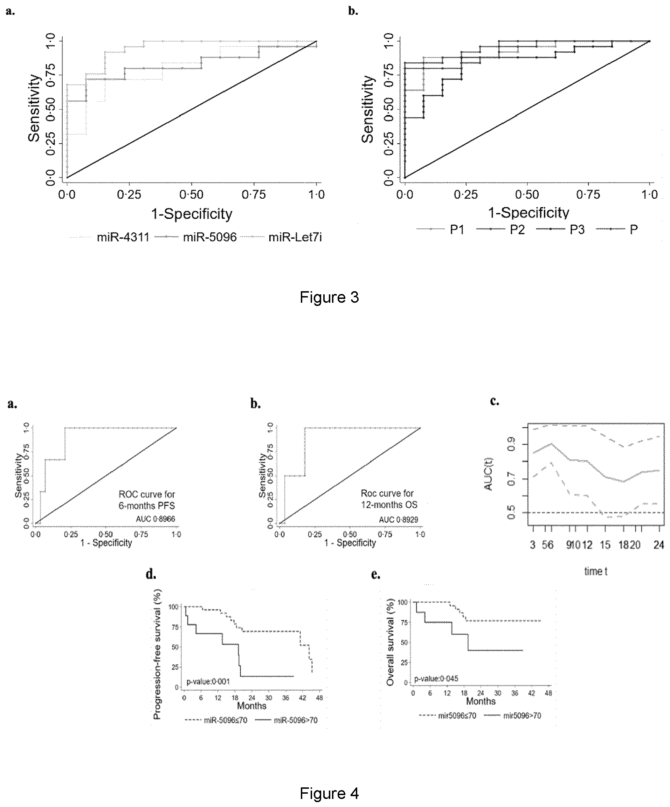

**Figure 3** **Circulating miRNAs signature predicts ¹⁸F-FDG/PET positivity in P-NET patients.** Performances of the circulating signature in predicting ¹⁸F-FDG/PET outcome in P-NET. (A) ROC curve of the single miRNAs (B) ROC curve of combined predictors, with significantly high AUCs. miR-4311: hsa-miR-4311; miR-5096: hsa-miR-5096; miR-let7i: hsa-let-7i-3p.
**Figure 4** **Hsa-mir-5096 can predict PFS and 12-mo OS in P-NET patients.** Performances of circulating hsa-mir-5096 in predicting PFS and 12-mo OS in P-NET patients treated with ¹⁷⁷Lu-DOTATATE. ROC curve analysis (a;b) of: (a) hsa-mir-5096 for 6-month PFS; (b) hsa-mir-5096 for 12-month OS. (c) Time dependent AUC curve (95% C.I) for hsa-mir-5096 prediction of 3-24-month PFS. Kaplan-Meier analysis (d-e) of: (d) hsa-mir-5096 for 6-month PFS in P-NET patients; (e) hsa-mir-5096 for 12-month OS in P-NET patients.

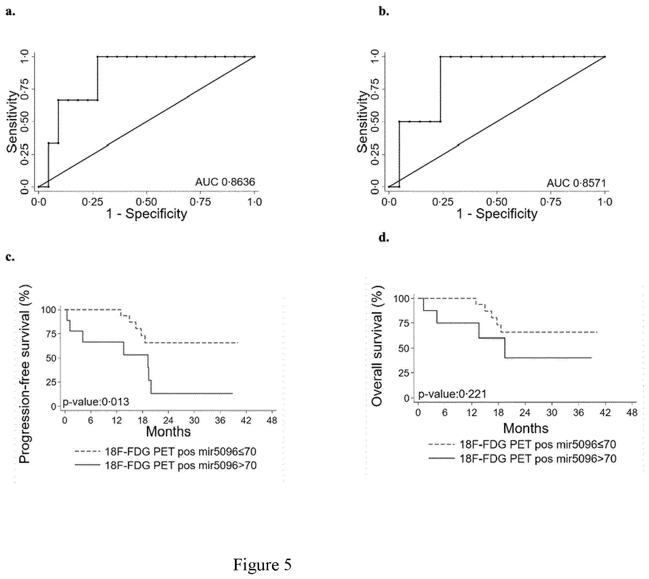

**Figure 5** **Hsa-mir-5096 can predict PFS and 12-mo OS in ¹⁸F-FDG/PET positive P-NET patients.** Performance of circulating hsa-mir-5096 in predicting 6-mo PFS and 12-mo OS in P-NET patients treated with ¹⁷⁷Lu-DOTATATE. ROC curve analysis (a;b): (a) hsa-mir-5096 for 6-month PFS in ¹⁸F-FDG/PET positive patients; (b) hsa-mir-5096 for 12-month OS in ¹⁸F-FDG/PET positive (+) subgroup. Kaplan-Meier analysis (c-d) of: (c) hsa-mir-5096 for 6-month PFS, in ¹⁸F-FDG/PET-CT positive subgroup; (d) hsa-mir-5096 for 12-month OS, in ¹⁸F-FDG/PET-CT positive subgroup.

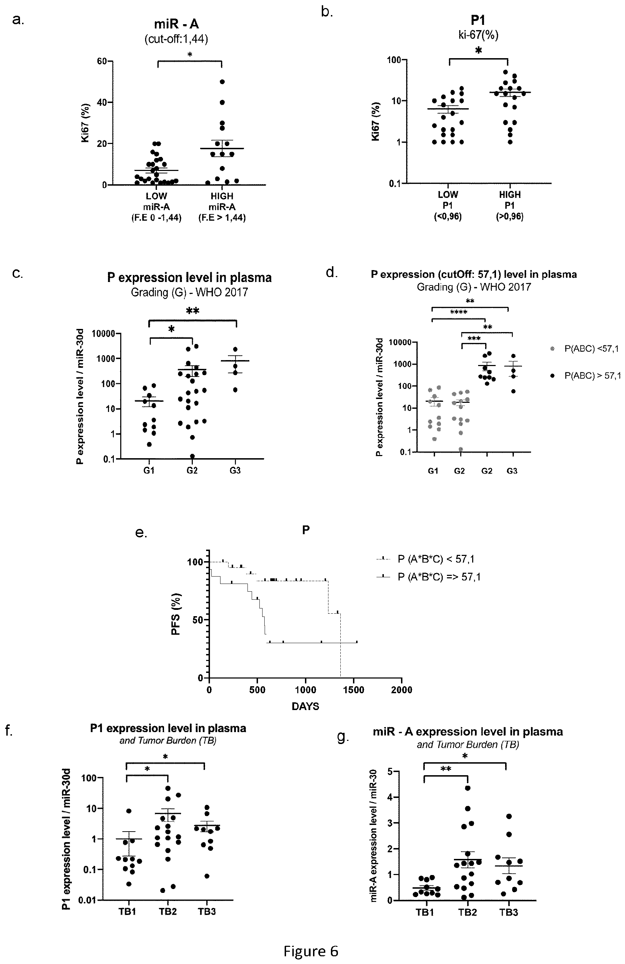

**Figure 6** **Hsa-miR-5096-5p can predict ki-67; Grading and Tumor Burden.** Hsa- miR-5096 correlation with clinical parameters: (a-b) ki-67(%); grading (c-e) and tumor burden (f-g) in P-NET.

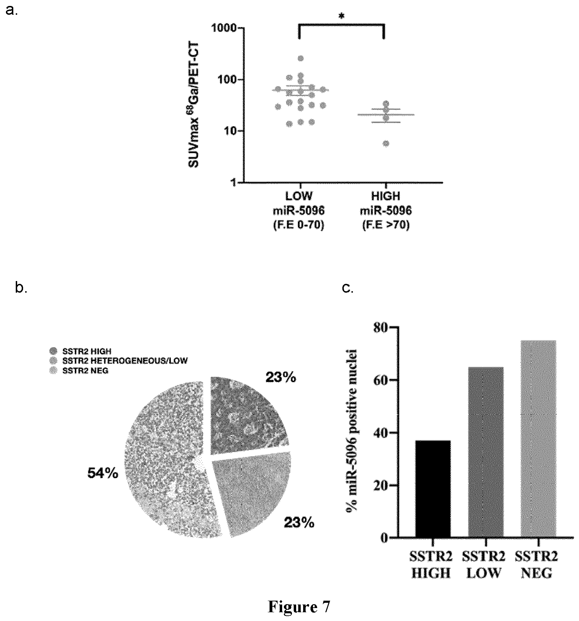

**Figure 7** **Hsa-miR-5096-5p overexpression inversely correlates with SSTR2 expression levels in P-NET patients.** (a) correlation analysis of expression of circulating hsa-mir-5096 and ⁶⁸Ga-PET SUVₘₐₓ in plasma. (b) Illustrative diagram of overall analyzed areas in terms of SSTR2 expression: frankly positive (23%), heterogeneous (23%) and negative (54%) expression areas; (c) Aggregated analysis of co-occurrence of hsa-mir-5096 positive nuclei (%) in overall areas with high, low and negative SSTR2 expression.

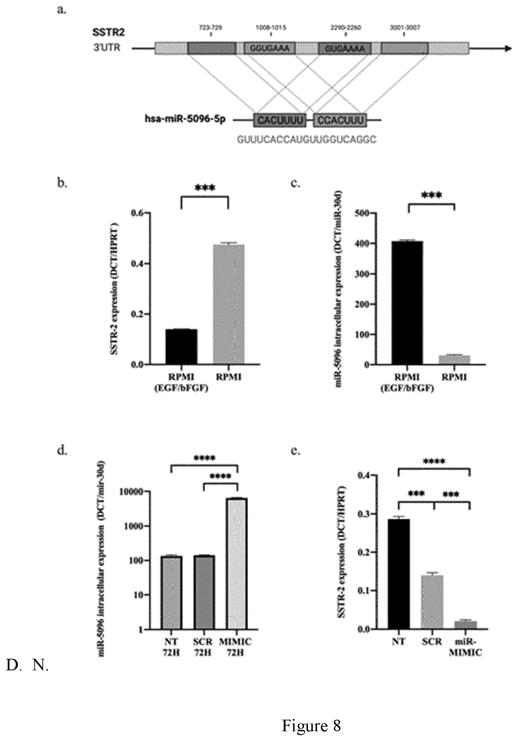

**Figure 8** **Hsa-miR-5096-5p overexpression down-modulates SSTR2 expression in NT-3 cell lines.** (a) Schematic representation of hsa-mir-5096 binding sites and their relative position on SSTR2 3'UTR; (b) hsa-miR-5096 basal expression level in NT-3 cell lines cultivated with or w/o growth factors (EGF; bFGF); (c) SSTR2 basal expression level in NT-3 cell lines cultivated with or w/o growth factors (EGF; bFGF); (d) hsa-miR-5096 and (e) SSTR2 expression in NT-3 cell lines 72h post transfection with miRCURY LNA miR-5096 mimic and scramble control.

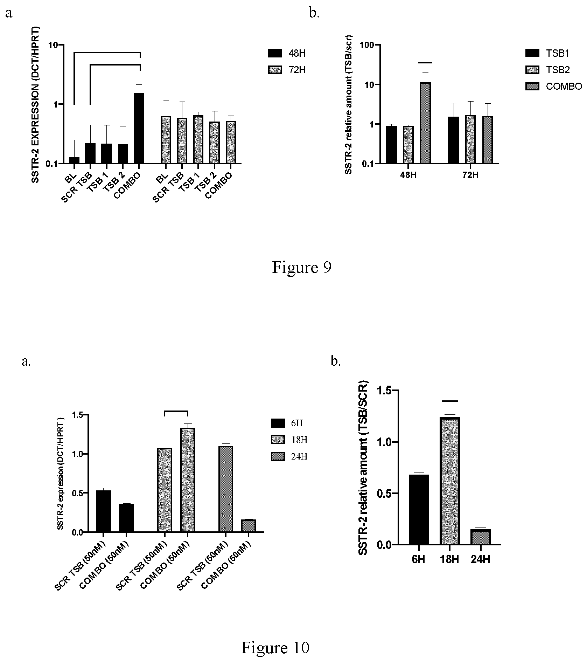

**Figure 9** **Inhibition of Hsa-miR-5096-5p binding on SSTR2 transcript up-regulates SSTR2 expression in NT-3 cell lines.** (a) SSTR2 expression level in NT-3 cell lines 48 and 72h post transfection with Target Site Blockers (TSB1 and 2 ), scramble control and combination (COMBO), data normalized via DCT on HPRT as housekeeping gene. (b) SSTR2 relative increase NT-3 cell lines 48 and 72h post transfection with Target Site Blockers (TSB1 and 2) alone and combination (COMBO) normalized via dDCT on scramble control as reference.
**Figure 10** **Hsa-miR-5096-5p inhibition via Target Site Blockers upregulates SSTR2 expression in NT-3 cell lines as early post-transcriptional events.** (a) SSTR2 expression level in NT-3 cell lines 6, 18 and 24h post transfection with TSB combination (COMBO) and scramble control at 50 nM concentration. Data were normalized via DCT on HPRT as housekeeping gene. (b) SSTR2 relative increase NT-3 cell lines 6, 18 and 24h post transfection with TSBs combination (COMBO) normalized via dDCT on scramble control as reference.

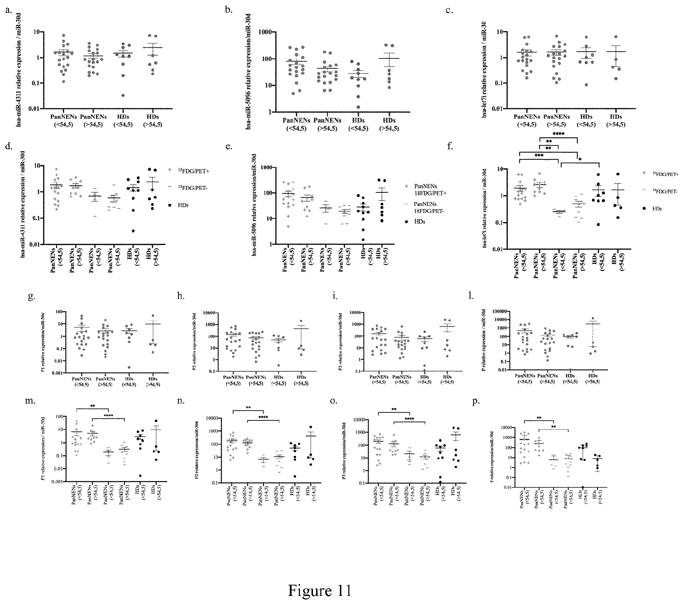

**Figure 11****. Age contribution to miR-signature and predictors expression level in plasma of PAN-NENs and Healthy Donors (cut-off: 54,5), according to 18F-FDG/PET.** Age contribution to single miR expression level in plasma of PAN-NENs and Healthy Donors (a-c): (a) hsa-miR-4311; (b) hsa-miR-5096; (c) hsa-let-7i-3p. Age contribution to single miR expression level in plasma of PAN-NENs and Healthy Donors, according to 18F-FDG/PET (e-g): (e) hsa-miR-4311, in 18F-FDG/PET positive and negative patients; (f) hsa-miR-5096, in 18F-FDG/PET positive and negative patients; (g) hsa-let-7i-3p,in 18F-FDG/PET positive and negative patients. Age contribution to predictors expression level in plasma of PAN-NENs and Healthy Donors, according to 18F-FDG/PET (m-p): (m) P1 (hsa-miR-4311^{∗} hsa-let-7i-3p), in 18F-FDG/PET positive and negative patients;; (h) P2 (hsa-mir-5096^{∗}hsa-let-7i-3p), in 18F-FDG/PET positive and negative patients;; (i) P3 (hsa-miR-4311^{∗} hsa-mir-5096), in 18F-FDG/PET positive and negative patients; (1) P (hsa-miR-4311^{∗}hsa-mir-5096^{∗}hsa-let-7i-3p), in ¹⁸F-FDG/PET positive and negative patients. Healthy Donors = HDs
**Figure 12****. Receiver Operating Characteristic (ROC) and Kaplan-Meier (KM) analysis of combined predictors (P1, P2, P3 and P) for 6-months progression free survival (PFS) and 12-months overall survival (OS).** The performance of circulating P2, P3 and P in predicting Progression Free Survival (PFS) outcome in PAN-NEN patients treated with 177Lu-DOTATATE (a-1). (a) Combined ROC curve of P2, P3 and P, with significantly high AUCs. Associated table reports AUCs values, the identified cut-off and sensitivity and specificity percentages of proposed biomarkers. (b-1) Kaplan-Meier analysis (KM) for Progression Free Survival (PFS) of: (b) P2 (cut-off: 123·3); (c) P2 (cut-off: 123·3), according to 18F-FDG/PET-CT outcome; (d) P2 (cut-off: 123·3), in 18F-FDG/PET-CT positive patients. (e) P3 (cut-off: 142·9) (f) KM analysis of P3 (cut-off: 142·9), according to 18F-FDG/PET-CT outcome; (g) P3 (cut-off: 142·9), in 18F-FDG/PET-CT positive subgroup; (h) P (cut-off: 108·3); (I) P (cut-off: 108·3), according to 18F-FDG/PET-CT outcome; (1) P (cut-off: 108·3), in 18F-FDG/PET-CT positive subgroup.

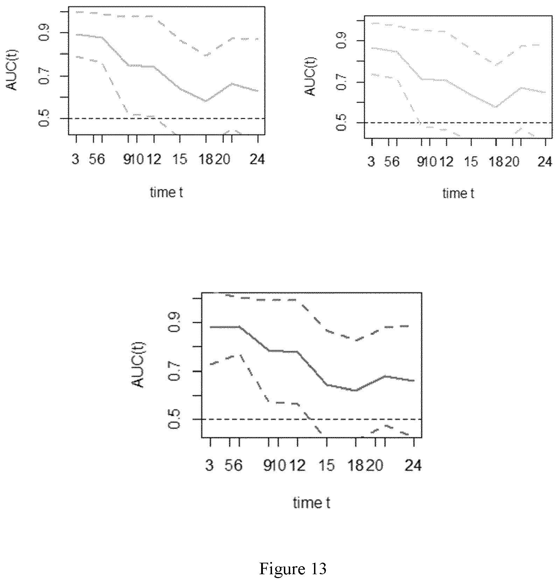

**Figure 13****. Time dependent AUC curve for P, P2 and P3.** Predictors P, P2 and P3 AUCS over time (range: 3-24 month) with 95% confidence interval (C.I)

### Definitions

Within the meaning of the present invention, for "at least one" it is intended one, two or more from the subsequent list, i.e. also combinations thereof.

Within the meaning of the present invention, for "blood sample" it is intended to be a sample of whole blood, plasma or serum or sample derived from the processing of those. In this context for "whole blood" it is intended the body fluid that delivers necessary substances such as nutrients and oxygen to the cells and transports metabolic waste products away from those same cells; typically it is composed of blood cells suspended in blood plasma. For "plasma" it is intended to be the liquid component of blood that is freed from blood cells. For "serum" it is intended plasma from which the clotting proteins have been removed.

Within the meaning of the present invention, for "normalization" it is intended the expression of a differential value in terms of a standard value to adjust for effects which arise from technical variation due for example to sample handling, sample preparation and measurement rather than biological variation of miRNA concentration in a sample.

Within the meaning of the present invention, for 18F-FDG-PET it is intended positron emission tomography (PET) with 2-deoxy-2-[fluorine-18]fluoro-D-glucose (18F-FDG), an analogue of glucose that provides valuable functional information based on the increased glucose uptake and glycolysis of cancer cells.

Within the meaning of the present invention, for 18F-FDG-PET positive lesion it is intended a lesion of cancer origin able to incorporate 18F-FDG and be visualized by PET imaging;

Within the meaning of the present invention, for Peptide Radionuclide Receptor Therapy (PRRT) it is intended a molecular targeted therapy used to treat neuroendocrine tumors (NET).

Within the meaning of the present invention, for overall survival (OS) it is intended the time which begins at diagnosis (or at the start of treatment) and up to the time of death of a subject.

Within the meaning of the present invention, for progression-free survival (PFS) it is intended the time from treatment initiation until disease progression.

Within the meaning of the present invention, for highly proliferating P-NET lesion it is intended a lesion which increases in size over time and express markers of active proliferation, such as ki-67, if assessed at the cellular level.

Within the meaning of the present invention, for tumor burden it is intended the number of cancer cells, the size of a tumor, or the amount of cancer in the body. For high tumor burden it is intended the presence of an elevated number of cancer cells or an large size of a tumor or a high amount of cancer in the body.

The miRNAs used in the methods of the invention are known and they have the following sequences:
hsa-miR-5096 - miRbase Accession number: MIMAT0020603;
Sequence: GUUUCACCAUGUUGGUCAGGC [SEQ ID N.1].
hsa-let-7i-3p - miRbase Accession number: MIMAT0000415;
Sequence: UGAGGUAGUAGUUUGUGCUGUU [SEQ ID N.2].
hsa-miR-4311 - miRbase Accession number: MIMAT0016863;
Sequence: GAAAGAGAGCUGAGUGUG [SEQ ID N.3].
hsa-miR-30d-3p - miRbase Accession number: MIMAT0004551
Sequence: CUUUCAGUCAGAUGUUUGCUGC [SEQ ID N.4]
hsa-miR-5096 and miR-5096 are herein used as synonyms. The same applies to the other mentioned miRNAs.

For miR-30d or hsa-miR-30d it is herein intended hsa-miR-30d-3p.

The method of the invention is carried out on a biological sample comprising blood obtained from said subject. In particular, it is carried out on a blood sample previously isolated from the subject.

In a preferred embodiment, the method is carried out on a plasma sample obtained by the whole blood sample for example through centrifugation.

Determination of the expression level of the miRNAs in a blood sample from a subject according to the methods of the invention can be carried out as explained below.

The blood sample previously obtained by a subject is collected in a collection sterile vessel containing ethylenediaminetetraacetic acid (EDTA) or similar chelating agents. Platelet free plasma is then generated from whole blood centrifugation or similar methods as known by the skilled in the art.

From the obtained platelet free plasma exosome-enriched fraction is isolated by means of known techniques such as: differential ultracentrifugation (dUC), ultrafiltration (UF), poly-Ethylene Glycol (PEG)-Based Precipitation, immunoaffinity capture, microfluidics, size-exclusion chromatography (SEC). See for example as a reference Sidhom K, Obi PO, Saleem A. A Review of Exosomal Isolation Methods: Is Size Exclusion Chromatography the Best Option? Int J Mol Sci. 2020 Sep 4;21(18):6466. doi: 10.3390/ijms21186466. PMID: 32899828; PMCID: PMC7556044 for detailed description of the methods.

Kits and products for exosome isolation are commercially available, for example Exoquick^{™}, SCBI, can be used. In an embodiment, one ml of plasma per sample is used for exosome-fraction enrichment via Exoquick^{™}, SCBI.

Small RNAs, including miRNAs, contained in the exosome-enriched fraction can be isolated by means of known methods or commercial kits. For example, MiRNeasy serum/plasma kit - Qiagen Cat No./ID: 217184 can be used.

Dosage of each specific miRNA can be performed by known methods. For example, quantitative PCR (RT/qPCR) can be used on frozen or fresh RNA samples on C1000 Touch Thermal Cycler (Bio rad^{™}, Hercules, CA, USA) or similar thermal cycler using TaqMan^{™} MicroRNA Reverse Transcription Kit (Applied Biosystems^{™}; Foster city, CA, USA. Cat No./ID: 4366596) or similar technologies. C-DNAs can be obtained by TaqMan^{™} MicroRNA or alternative Reverse Transcription protocol for the single miRNAs or by multiplexing miRNA primers for the target miRNA. Target miRNAs can be also detected individually using universal Master Mix and TaqMan^{™} miRNA Assay or any validated miRNA specific probes, according to the manufacturer's protocol (for example Applied Biosystems^{™}, Foster city, CA, USA. Cat No./ID: 4440040). RT/qPCR analysis can be conducted using Applied Biosystems^{™} 7500 Real-Time PCR Systems (Applied Biosystems^{™}; Cat No./ID: 4351104).

See Dharmawardana, N., Ooi, E. H., Woods, C., & Hussey, D. (2019). Circulating microRNAs in head and neck cancer: a scoping review of methods. Clinical & Experimental Metastasis. doi:10.1007/s10585-019-09961-6 for exemplary detailed methods to obtain miRNAs from plasma specimens.

Determination of the expression level of a reference miRNA, RNA, DNA or other biomolecule which does not change its amount in patients of interest with respect to a healthy subject can be carried out according to the general knowledge in the field. The expression level of the reference molecule is determined in the same blood sample on which the expression level of the target miRNAs is determined. The reference molecule can be selected by the skilled person according to the common general knowledge in the field, in particular it can be selected among the miRNAs, RNAs, DNAs or other biomolecules which are known to not change their amount in a subject affected by a GEP tumor with respect to a healthy subject. In a preferred embodiment the reference molecule is a miRNA, such as the so called housekeeping miRNAs. Preferably, it is hsa-miR-30d.

Determination of the expression level of a reference miRNA, for example hsa-miR-30d, can be carried out as described in the above paragraphs. The expression level of the reference miRNA is determined in the same blood sample on which the expression level of the target miRNAs is determined.

Normalization of the expression levels of single target miRNAs on the expression of the reference molecule, such as housekeeping miRNA, can be carried out as known in the field.

For example, the fold enrichment is obtained by means of the 2-ΔCT method for the corresponding sample. According to said method for example the fold enrichment can be calculated according to the following formula: 2exp-[Ct(miRNAx)-Ct(miRNAref)]. "Predictors" (P1, P2, P3 and P) are created as the product of fold enrichments of single miRNAs.

Based on the chosen reference molecule, cut-off values for each application can be calculated.

In a first aspect, the present invention provides a method for differential diagnosis of NETs of pancreatic origin (P-NETS) and of small-intestine origin (SI-NETS), as described above.

In an embodiment, the subject in need thereof is a subject diagnosed with GEP-NET or having at least one symptom of GEP-NET or having a familiar history or a known predisposition for GEP-NET.

In a second aspect, a method for diagnosing an ileal primary site of origin in a subject affected by Small Intestine NET (SI-NET) is provided, as described above.

For ileal primary site of origin it is intended that the cancer arises from the ileum tissue, the final tract of the small intestine.

The diagnosis of an ileal primary site of origin through liquid biopsy is advantageous because it allows a better definition of the type of neuroendocrine cancer of the patient without the need for an invasive procedure.

In a third aspect, a method to distinguish 18F-FDG-PET positive from negative P-NET patients, the latter characterized by less aggressive disease and better prognosis after PRRT is provided, as disclosed above.

In particular, highest predictivity of 18F-FDG-PET positive lesions is reached by combination of hsa-let-7i-3p and hsa-miR-5096.

In a fourth aspect, the invention provides a method to predict 6 month progression-free survival (PFS) in a subject affected by a pancreatic neuroendocrine tumor (P-NET), in particular who is undergoing a treatment targeting somatostatin receptors, more preferably a treatment with Peptide Radionuclide Receptor Therapy (PRRT), as described above.

In an embodiment, the subject affected by a pancreatic neuroendocrine tumor (P-NET) is undergoing a treatment with Peptide Radionuclide Receptor Therapy (PRRT) and is positive to ¹⁸F-FDG-PET. In this embodiment, a normalized expression level of hsa-miR-5096 in the sample equal or greater than 70 identifies a subset of 18F-FDG-PET positive patients that progress even earlier than six months and do not benefit from PRRT treatment.

In particular embodiments, the normalized expression level of hsa-miR-5096 or of the following combinations of miRNAs: hsa-mir-5096 and hsa-let-7i-3p, hsa-miR-4311 and hsa-mir-5096 and hsa-miR-4311, hsa-mir-5096 and hsa-let-7i-3p is evaluated. Expression levels above the indicated cut-offs indicate patients with shorter PFS, with significant AUCs. In particular, highest predictivity of 6-month PFS is provided by hsa-miR-5096 alone. Indeed, expression level of hsa-miR-5096 greater than 70 best predicts P-NET patients with shorter PFS within 6-month. hsa-miR-5096 can be used as a prognostic tool for monitoring PFS of patients up to 24 months since it maintains significant AUC values.

In a fifth aspect, it is provided a method to predict 12 month overall survival (OS) in a subject affected by a pancreatic neuroendocrine tumor (P-NET), preferably who is undergoing a treatment targeting somatostatin receptors, more preferably a treatment with Peptide Radionuclide Receptor Therapy (PRRT), as disclosed above.

A normalized expression level of hsa-miR-5096 in the sample equal or greater than 70 best predicts P-NET patients with shorter OS with significant AUC.

In a sixth aspect, it is provided a method for predicting the presence of highly proliferating P-NET lesions in a subject affected by a pancreatic neuroendocrine tumor (P-NET), as defined above.

In an embodiment, a normalized expression level of single hsa-miR-4311 or hsa-let-7i-3p or of their combination above the identified cut-offs significantly correlates with higher ki-67 (%) in P-NET patients with significant AUCs (see Table 3 and Figure 6 a-b). Ki-67 is a known marker for proliferating cells and it is therefore an indicator of a highly proliferating tumor lesion. Expression level of hsa-miR-4311 greater than 1,44 best predicts P-NET with higher proliferating tumor.

In a seventh aspect, it is provided a method for grading a pancreatic neuroendocrine tumor (P-NET) in the highest grade G3, as defined above. The WHO classification categorizes NET as a NET (3) grade 1, NET grade 2, and NEC grade 3. The method of the invention allows to grade a P-NET tumor as a G3.

In an embodiment, a normalized expression level of combination of hsa-miR-4311, hsa-miR-5096 and hsa-let-7i-3p (also named predictor P) in the sample obtained from a patient affected by P-NET equal or greater that 57.1 predicts a G3 P-NET with significant AUC of 0,83 and 100% specificity and 65% sensitivity (Figure 6 c-e).

In an eighth aspect, is provided a method for predicting tumor burden in a subject affected by a pancreatic neuroendocrine tumor (P-NET), as defined above.

In an embodiment, normalized expression levels of hsa-miR-4311, alone or in combination with hsa-let-7i-3p in the sample obtained from a patient affected by P-NET above identified cut-offs indicate a high tumor burden compared to low tumor burden P-NET patients (Figure 6 f-g).

In a ninth aspect, it is provided a method to predict response to therapy in a subject affected by a pancreatic neuroendocrine tumor (P-NET), as defined above. In an embodiment, a positive response to therapy is predicted if hsa-miR-5096 normalized expression is below the cutoff value of 70. For positive response to therapy is intended for example that the subject that undergoes the treatment has a longer life expectation than if he would not be treated. Treatment can be for example with PRRT.

In an embodiment, normalized expression level of hsa-miR-5096 in the sample greater than 70. predicts low Ga uptake (SUVₘₐₓ ) at ⁶⁸Ga-DOTA-PET/CT scan, identifying patients who will not benefit from PRRT.

It is a further object of the invention an inhibitor of hsa-miR-5096 for use for the treatment of Gastro-entero-pancreatic neuroendocrine tumors (GEP-NETs). Preferably, said inhibitor is for use for the treatment of aggressive pancreatic neuroendocrine tumor (P-NET). More preferably said P-NET has low to heterogenous expression of somatostatin receptor SSTR2. In a preferred embodiment, said inhibitor is able to inhibit the binding of hsa-miR-5096 on SSTR-2 transcript.

Within the context of the present invention, the term "inhibitor" means an agent capable of decreasing or blocking, completely or partially, the activity of the target miRNA, i.e. hsa-miR-5096. In particular, it means an agent capable of sufficiently decreasing the activity of hsa-miR-5096. In a particular embodiment, it means an agent capable of decreasing or blocking the interaction of hsa-miR-5096 with the somatostatin receptor SSTR-2 transcript or to decrease hsa-miR-5096 expression.

SSTR-2 transcript is available on the GenBank database: NCBI Gene ID: 6752; GenBank Accession:NM_001050.03.

Said inhibitor can be an oligonucleotide comprising at least 5 nucleotides capable of binding to a hsa-miR-5096 by complementary base pairing, thereby decreasing or blocking the function of said miRNA. For "complementary base pairing" it is intended that some or all of the bases of said oligonucleotide pair with some or all of the bases of said miRNA target. In particular, said inhibitor can be able to pair at least 40% of its bases with the bases of the target miRNA; for example said inhibitor is able to pair at least 40%, 50%, 60%, 70%, 80%, 90% or 100% of its bases with the bases of the target miRNA.

In a preferred embodiment, the inhibitor is chosen between a locked-nucleic acid (LNA)-based oligonucleotide and an anti-miR.

In an embodiment, the inhibitor is an anti-miR wherein said anti-miR is an RNA oligonucleotide at least partially complementary to the target miRNA. In particular, it is sufficiently complementary to bind and at least partially block said target miRNA. Preferably, said anti-miR comprises between 5 and 27 nucleotides and it has at least 40% of the bases complementary with the bases of the target miRNA. Within the context of the present invention, the term "anti-miR" or "antagomir" means an oligonucleotide molecule that prevents the binding of other molecules to one or more specific miRNAs.

In a preferred embodiment, said inhibitor is an LNA-based oligonucleotide. Preferably, it comprises between 5 and 27 nucleotides and it comprises at least one locked nucleic acid. It can also comprise more than one locked nucleic acid. Said LNA-based oligonucleotide is at least partially complementary to the miRNA target, in particular it can be at least 40%, 50%, 60%, 70%, 80%, 90% or 100% complementary to the miRNA target.

Within the context of the present invention, the term "locked nucleic acid" or "LNA" means a nucleotide with the ribose ring blocked in an N-type conformation by a 2'-O, 4'-C bridge. LNAs are described for example in WO 99/14226, WO 00/56746, WO 00/56748, WO 01/25248, WO 02/28875, WO 03/006475 and WO 03/095467 and the references mentioned therein.

Within the context of the present invention, the term "LNA-based oligonucleotide" means an oligonucleotide comprising at least one locked nucleic acid (LNA), as defined above. This oligonucleotide is generally a small synthetic RNA at least partially complementary to the target miRNA, in particular sufficiently complementary to bind and block the target miRNA, including at least one locked nucleic acid.

LNA-based oligonucleotides are inhibitors of common use in the field and commercially available. For example, they are available from the following companies: QIAgen, Affymetrix, Perkin Elmer.

In a particular embodiment of the invention, said LNA-based oligonucleotide is an oligonucleotide, in particular an antisense oligonucleotide, with perfect sequence complementary to the miRNA target. Said LNA-based oligonucleotide can be 100% complementary to the miRNA target. For example, it can be an LNA-based oligonucleotide with perfect sequence complementary to the miRNA target of the type miRCURY LNA miRNA Power Inhibitor, commercially available from QIAgen.

The expert in the field is able to find an inhibitor suitable for use according to this invention according to the general knowledge in the field.

Indeed, it is well known in the field how to identify an agent able to inhibit the activity of a known miRNA. In particular, anti-miR and LNA-based oligonucleotides are commonly used to inhibit the activity of target miRNAs. A skilled person in the field can design or purchase anti-miR or LNA-based oligonucleotides able to inhibit the activity of hsa-miR-5096.

In a preferred embodiment, said inhibitor is able to bind and inhibit at least one of the two following sequences on miR-5096:
CACUUU (SSTR2_5096_1310)
CCACUUU (SSTR2_5096_1025)

These sequences recognize the following sequences GUGAAAA; GGUGAAA located on 4 sites at the 3'-UTR of the SSTR-2 gene at position 723-729 and 3001-3007 / 1008-1015 and 2290-2260, respectively.

In an embodiment, said inhibitor is an agent capable of decreasing or blocking the interaction of hsa-miR-5096 with the somatostatin receptor SSTR-2 transcript.

Such inhibitor can for example bind and block one or more of the binding sites of hsa-miR-5096 on the SSTR-2 transcript.

In an embodiment, said binding sites of hsa-miR-5096 on the SSTR-2 transcript have the sequences GUGAAAA; GGUGAAA located at the 3'-UTR of the SSTR-2 gene at positions 723-729 and 3001-3007 / 1008-1015 and 2290-2260, respectively.

Preferably, the inhibitor targets all said 4 binding sites on the SSTR-2 transcript.

In a preferred embodiment, said inhibitor is a LNA Target Site Blocker (TSB) able to bind and block one or more of said binding sites on the SSTR-2 transcript

Suitable LNA Target Site Blockers (TSB) are commercially available or can be manufactured according to the common knowledge in the field.

The inventors found out that the use of target site blockers aimed to shield specific sequences targeted by hsa-miR5096 on the SSTR-2 3'-UTR allowed SSTR-2 mRNA overexpression in NT-3 cells.

The inhibitor for use as described in the present invention may be administered to a subject in need thereof in any form. In particular, it may be administered by conventional methods of administration of small RNAs.

In a preferred embodiment, it is administered encapsulated in nanoparticles, according to conventional methods in the field.

In another preferred embodiment, it is administered via specific armed cells (e.g B- lymphocytes)

The cells or nanoparticles can be modified in order to be able to bind to GEP-NET tumor cells, for example by decorating the particles with specific molecules (i.e. small molecules, peptides, antibodies).

In an embodiment, the inhibitor is administered without the use of transfection agents.

In an alternative embodiment, it is administered in a pharmaceutical composition.

The expert in the field will decide the effective timing of administration, depending on the patient's condition, the level of severity of the pathology, the patient's response and any other clinical parameters included in the general knowledge in the field.

According to the present invention, the inhibitor can be administered together with lipid molecules, such as cationic lipids that can facilitate its transport, according to the state of the art. A further method of administering such an inhibitor is by means of a suitable vector, known for the administration of RNA or DNA. A preferred vector is the adeno-associated vector (AAV), a viral vector well known for in vivo administration of DNA (Mingozzi F, High KA: Therapeutic in vivo gene transfer for genetic disease using AAV: progress and challenges. Nature reviews genetics. 2011 May;12(5):341).

Injection is a preferred route of administration. Injection can be systemic or directly at the tumor site. An expert in the field may decide to administer the inhibitor by any conventional route.

For general knowledge in the field, reference can be made to Remington's Pharmaceutical Sciences, latest edition.

It is also an object of the invention, a kit useful for performing the methods above described.

The kit may for example contain one or more of the followings:
- agents and tools to obtain plasma exosome-enriched fraction from a blood sample previously isolated from a subject;
- agents and tools to isolate target miRNAs;
- agents and tools for the dosage of each specific miRNA, for example primers and probes specific for the target miRNAs and/or useful for RT-PCR. The agents can be polynucleotides or other molecules which specifically bind to or specifically hybridize to the target miRNAs. The agents include polynucleotides, such as probes and primers, e.g. sense and antisense PCR primers, having identity or complementarity to the target miRNAs.

It is also an object of the invention, a device for carrying out the methods above disclosed.

Such device comprises agents and tools for carrying out each of the steps of each method.

For example it can be a device based on PCR amplification or able to directly recognize and quantify the target miRNAs.

The invention will be now illustrated by the following examples.

### EXAMPLES

### Materials & Methods

### Patients' characteristics

From October 2016 to September 2019, a cohort of 68 histologically or cytologically confirmed G1, G2 and G3 GEP - NET patients (age >18 years, both genders) with RECIST based progressive disease (PD), has been enrolled in the clinical trials NCT02736500 (LUX) and NCT02489604 (LUNET).

According to inclusion criteria all included patients displayed appropriate hematological, liver and renal parameters (hemoglobin >= 10 g/dL; absolute neutrophil count (ANC) >= 1.5 × 109 /L; platelets >= 100 × 109 /L; bilirubin ≤1.5 X UNL (upper normal limit), ALT <2.5 X UNL (< 5 X UNL in presence of liver metastases), creatinine < 2 mg/dL). Eligible patients did not receive other treatments (e.g., chemo- or radiotherapy) from one month before to two months after the completion of 177Lu-DOTATATE cycles. Patients were naive from previous radionuclide treatments with radiopeptides (e.g., ¹¹¹Inpentetreotide, ⁹⁰Y-DOTATOC) or other radiopharmaceuticals (e.g., ¹³¹I-MIBG, ¹³¹I). Patients' disease was measurable by means of conventional imaging (CT or MRI). From this cohort, a homogeneous subset of 24 GEP -NET patients were randomly selected according to ¹⁸F - FDG/PET outcome (12 ¹⁸F - FDG/PET *positive* and 12 ¹⁸F - FDG/PET *negative)* for whole miRNome NGS profiling. In particular, 6 out of 24 GEP-NET from the training cohort were P-NET while 18 out of 24 were SI-NETs. Overall case series of 68 GEP-NET (37 ¹⁸F - FDG/PET *positive* and 31 ¹⁸F - FDG/PET *negative)* patients was considered as a validation set for RT/qPCR downstream validation. In particular, 30 out of 68 were SI-NETs and 38 out of 68 were P-NETs. All patients provided a signed informed consent for the blood withdrawal, prior to ¹⁷⁷Lu-DOTATATE PRRT and downstream genomic analysis. An additional cohort of 17 healthy donors was considered for blood withdrawal and subsequent molecular comparison with P-NET.

This study was approved by the local ethical committee (CEROM), approval no. 6711/5.1/2016, and performed according to Good Clinical Practice standards and the Declaration of Helsinki.

Statistical analysis of the population distribution is provided in Table 4. Statistical analysis of age contribution to miR-signature and predictors predictivity of P-NETs and Healthy Donors, according to ¹⁸F-FDG/PET is described in Figure 11.

All statistical analyses were performed using Stata/SE version 15.1 for Windows (StataCorpLP, College Station, TX, USA). TimeROC R package was used to plot time-dependent AUC curve and 95% confidence interval.

### Plasma specimen's collection

Blood samples from the *overall case series* were collected by venipuncture at baseline, prior to ¹⁷⁷Lu-DOTATATE PRRT. Blood was collected in a 3 mL K3-EDTA collection sterile vessel. Whole blood was centrifuged at 2500g for 10 minutes at room temperatures to obtain *platelet free* plasma. Plasma was carefully transferred into new 15 mL conical tubes (Falcon ^{™}) for a second centrifugation at 2500× g for 10 min to remove further cellular debris. At least 1 ml of supernatant was collected and stored at -80°C until required.

### Small - RNA exosome - enriched fraction precipitation

Thawed, frozen plasma samples were precipitated using Exoquick^{™}, SCBI according to the manufacturer's protocol to obtain exosome-enriched fraction small-RNAs. Exoquick^{™}, SCBI allows the precipitation of 20-100 nm vesicles and to extract their content. The pellet containing exosome-enriched fraction RNAs was resuspended in 200 ul of sterile PBS (1X). Qiazol^{™} was added to provide cryopreservation and lysis for exosome associated miRNA extraction.

Small RNAs, including miRNAs, were isolated with miRNeasy serum/plasma kit (Qiagen Cat No./ID: 217184) according to the manufacturer's protocol.

One ml of plasma per sample was used and RNA eluted in 56 ul of RNase-free water.

### Whole miRnome Next Generation Sequencing (NGS) profiling and analysis

Specimens from 24 GEP -NET patients were selected according to ¹⁸F - FDG/PET outcome (12 ¹⁸F - FDG/PET *positive* and 12 ¹⁸F - FDG/PET *negative)* for whole miRNome NGS profiling.

Small RNA transcripts were converted into barcoded cDNA libraries. Library preparation was created with the NEBNext Multiplex Small RNA Library Prep Set for Illumina (New England BioLabs Inc., USA). Libraries were pooled in 24 samples and run on Illumina NextSeq 550 platform, 2X75 cycles (Illumina, USA). The obtained BCL Files were converted to FASTQ Files and data quality was assessed by FastQC software. Reads shorter than 14 nucleotides were discarded from the analysis; the remaining reads were trimmed from the adapter sequences using Cutadapt software (http://journal.embnet.org/index.php/embnetjournal/article/view/200). The trimmed reads were mapped against the precursor miRNA sequences downloaded from miRBase (Release 21) by the Shrimp algorithm. A matrix of integer values called counting matrices was created. DESeq2 Bioconductor's package was used to identify the expression of miRNAs in the different groups. Endogenous controls for RT/qPCR were selected from the NGS data by considering the following criteria for each raw data: at least 5 reads for each sample and a log2 standard deviation value < 16.

### RT/qPCR

Candidate miRNAs emerged from whole miRnome NGS profiling were validated by quantitative PCR (RT/qPCR) on the same samples and on an independent case series (n. 68). C-DNAs from frozen and thawed RNA were obtained on C1000 Touch Thermal Cycler (Bio rad^{™}, Hercules, CA, USA); using TaqMan^{™} MicroRNA Reverse Transcription Kit (Applied Biosystems^{™}; Foster city, CA, USA. Cat No./ID: 4366596), cycling conditions were set according to the manufacturer's protocol. TaqMan^{™} MicroRNA Reverse Transcription protocol was optimized multiplexing the TaqMan^{®} miRNA Assay's primers for the following targets: hsa-miR-3133, hsa-miR-4311, hsa-miR-5096, hsa-let-7i-3p normalized with hsa-miR-30d as reference housekeeping miRNA (multiplexing group 1); hsa-miR-519c-3p, hsa-miR-582-3, hsa-miR-3614-5p, hsa-miR-1246 and miR-423-3p as reference housekeeping miRNA (multiplexing group 2).

Universal Master Mix without UNG and TaqMan^{™} miRNA Assay specific probes, for each target miRNA were used according to the manufacturer's protocol (Applied Biosystems^{™}, Foster city, CA, USA. Cat No./ID: 4440040). RT/qPCR analysis was conducted using Applied Biosystems^{™} 7500 Real-Time PCR Systems (Applied Biosystems^{™}; Cat No./ID: 4351104).

Expression level of single target miRNAs was normalized to hsa-miR-30d and the fold enrichment was obtained by means of the 2^{-ΔCT} method, for the corresponding sample. In addition, "predictors" (P1, P2, P3 and P) were created as the product of fold enrichments (2^{-ΔCT}) of single miRNAs, to improve single targets and prognostic power.

### Statistical analysis

Categorical data were expressed as absolute numbers and percentage, while continuous variables were shown as median and range. ¹⁸F - FDG/PET positive and negative miRNAs and predictors median expression level (2^{-ΔCT}) were compared. Normality of distribution of continuous data was assessed through the Shapiro-Wilk test. Wilcoxon and Mann-Whitney test, chi-square test were applied. To evaluate relations among two continuous variables, Pearson's correlation coefficient was applied.

The Receiver Operating Characteristic (ROC) curve, which is defined as a plot of sensitivity vs 1-specificity, was performed as evaluation of the performance of some of three miR-signatures and their combination to predict PET positivity, 6-month PFS and 12-months OS. AUC (with 95% confidence - CI) was calculated as a common measure of accuracy and values range from 0.5 to 1.0: higher values are corresponding to a better performance of tested values. AUC values higher than 0.7 were considered as acceptable values.

OS was calculated as the time from date of start PRRT therapy to date of death or last follow-up visit, while PFS was calculated at 6 - month. Alive patients were censored at last visit while patients without disease progression were censored at last tumor evaluation. Kaplan Meier (KM) curves were used to estimate the survival function and the Log-rank test was used to compare different subgroups in terms of OS or PFS. Median OS and median PFS were calculated, and 95% confidence intervals (95%CI) were reported.

### Immuno-miRNA-ISH

Study protocol was amended to allow the collection of histologically confirmed G1, G2 and G3 P-NET specimens in order to evaluate the hsa-miR-5096 and SSTR2 relative expression at the tissue level. A novel semi-automated combined IHC and miRNAs-ISH protocol was developed for the simultaneous detection of SSTR2 protein and hsa-miR-5096 miRNA expression.

MiRCURY LNA miRNA Detection probe for hsa-miR-5096, U6 small nuclear, *positive control* probe (Qiagen, Valencia, CA; Cat No./ID: 99002-15) and the scramble negative control probe (Qiagen, Valencia, CA Cat No./ID: 99004-15), were used. Each probe was labelled 5'3'DIG. Before starting, double-DIG-LNA probes were denatured by heating (90°C for 4 min) and then diluted to 50 nM in the ISH buffer (miRCURY LNA miRNA ISH Buffer Set-FFPE).

The first phase (tissue preparation, permeabilization and hybridization) has been performed in manual mode according to the miRCURY LNA miRNA detection probe protocol, while the second phase (signal detection) is automated using the OptiviewDAB Detection Kit (Ventana Medical Systems, Tucson, Arizona, USA) on Ventana BenchMark ULTRA (Ventana Medical Systems). The automated protocol has been set considering pre-treatment, hybridization and stringency washing steps. Automated detection includes endogenous peroxidase blocking, casein blocking (16 min), incubation (37°C for 1 h) with primary prediluted mouse anti-DIG antibody (Ventana Medical Systems), and signal detection with OptiviewDAB Detection Kit (Ventana Medical Systems), consisting of HQ Universal Linker incubation (for 12 min), HRP Multimer incubation (for 12 min), Optiview Amplification Kit detection steps (12 min each). The double staining for SSTR2 expression was performed straight forward on Ventana BenchMark ULTRA after cell conditioning with ULTRA CC1 (Ventana Medical Systems) for 24 min and casein blocking, using the antibody anti-SSTR2 (UMB1-C Terminal-ab134152-Abcam) in Ventana antibody diluent, incubated (37°C for 1 h), detected with Ultraview Universal Alkaline Phosphatase Red Detection Kit (Ventana Medical Systems).

Finally, slides were counterstained with Haematoxylin II (Ventana Medical Systems), washed in tap water with soap in order to eliminate the coverslip oil, dehydrated in the stove and and mounted with xylene and EUKITT mounting medium (Sigma-Aldrich, Merck KGaA, Darmstadt, Germany).

All the images (20 fields/sample) were acquired using a Red-Green-Blue (RGB) 24-bit camera, embedded in an optical widefield microscope equipped with a 20X objective.

### AND-Tool software interface development

To analyze the marker expression of the single nuclei in the histological samples, we designed a user-friendly freely available Graphical User Interface (GUI) requiring a minimal user interaction. The GUI has been named *Analysis Nuclei DAB (AND)-Tool* and it allows to automatically segment the nuclei and extract intensity/morphological features at the single-nuclei level. The *AND-Tool* was created using Matlab (The MathWorks, Inc., Massachusetts, USA). Source code, standalone execuTable version, documentation, and sample images are available for download from: https://sourceforge.net/p/andtool/.

First, all the acquired RGB images were corrected for uneven illumination by subtracting the background estimated with the standard ImageJ/Fiji rolling ball algorithm. Then, the RGB images were unmixed using the Colour Deconvolution ImageJ/Fiji plugin imposing the "FastRed-FastBlue-DAB" modality. The FastRed channel was used to subdivide the field of view into three distinguished types of regions of interest (ROIs, i.e., dark-red, light-pink and white ROIs) according to the local intensity and two fixed thresholds (hereafter named Th1 and Th2, with Th1 lower than Th2), manually defined from the user just once for all the images to be analyzed. The "dark-red" ROIs are those regions with intensity values of the FastRed channel between 0 and Th1; the "light-pink" ROIs, with intensity values between Th1 and Th2; the "white" ROIs, with intensity values between Th2 and 255. Nuclei have been detected using the FastBlue and the DAB channels. To detect the nuclei, we used an intensity-based k-mean classifier automatically subdividing the single channels into three regions: white background, weak cytoplasmic signal, and nuclear signal. The standard watershed segmentation algorithm was then used to analyze the nuclear signal and split touching objects to proceed in a single-nuclei analysis. Objects with size not compliant with that of a nucleus were filtered out to compute the masks of the real nuclei.

Single-nuclei intensity/morphological features and region-based statistics were computed using the intensity maps created by subdividing the sample areas in dark-red, light-pink and white ROIs. Two types of nuclei have been considered: the ones positive for the DAB staining, and the ones positive for the FastBlue staining but not positive for DAB

### Cell culture

Hsa-miR-5096 and SSTR2 expression were assessed in NT-3 cell lines³⁰ cultivated in RPMI medium supplemented with 10% FCS, penicillin/streptomycin, HEPES both with EGF (20 ng/mL; PreproTech, Rocky Hill, New Jersey), and FGF2 (10 ng/mL; PreproTech, Rocky Hill, New Jersey) and without growth factors (bFGF; EGF). NT-3 cells were cultivated in culture dishes coated with collagen type IV from Human Placenta (Sigma-Aldrich, Homefield Road, Haverhill, UK; Cat No./ID: 27663).

### Hsa-miR-5096 mimic transfection

To evaluate SSTR2 downmodulation in NT-3 cell lines, 3 × 10⁵ cells were plated into 6-well dishes coated with collagen type IV from Human Placenta (Sigma-Aldrich, Homefield Road, Haverhill, UK; Cat No./ID: 27663). After 24 hours, 15 and 30 pmol of hsa-miR-5096 miRCURY LNA miRNA Mimic and Scramble (Qiagen, Valencia, CA) were transfected using RNAiMAX transfection reagent (Invitrogen^{®}, Carlsbad, CA, USA) according to the manufacturer's instructions. Cells and culture medium were collected after 24h and 72h transfection. A Fixed volume of 700 ul of Trizol^{®} reagent has been added to dried pellets and miRNeasy Mini Kit 50 (Qiagen, Valencia, CA Cat No./ID: 217004) was used for RNA extraction to quantify hsa-miR-5096 and SSTR2 RNA levels. Concurrently, the culture medium was collected, and the exosome-enriched fraction was precipitated using Exoquick^{™} protocol for cultured cells (SCBI Cat No./ID: EXOTC50A-1). MiRNAs contained in the exosome-enriched fraction were extracted using miRNeasy serum/plasma kit (Qiagen, Valencia, CA; Cat No./ID: 217184), according to the manufacturer's protocol.

SSTR2 expression in NT-3 cell lines was assessed by RT/qPCR, after 24h and 72h mimic transfection. Expression values were expressed as (Ct) values normalized to the housekeeping gene HPRT (2^{-ΔCT} method) and then normalized to the corresponding scramble-control using the 2^{-ΔΔCT} method. Transfection efficacy and statistical significance were assessed by parametric t-test, comparing expression median value (+/-SD).

### P-NEN and non-NEN cell lines culturing

P-NEN (NT-3 and BON-1) and a non-NEN cell line (CAPAN-1). NT-3 were cultivated in RPMI medium supplemented with 10% FCS, penicillin/streptomycin, HEPES with EGF (20 ng/mL; PreproTech, Rocky Hill, New Jersey), and FGF2 (10 ng/mL; PreproTech, Rocky Hill, New Jersey) and without growth factors (bFGF; EGF). NT-3 cells were cultivated in culture dishes coated with collagen type IV from Human Placenta (Sigma-Aldrich, Homefield Road, Haverhill, UK; Cat No./ID: 27663). BON-1, were cultivated in DMEM medium supplemented with 10% FCS, penicillin/streptomycin and L-glutamine while CAPAN-1 were cultivated in IMDM medium supplemented with 20% FCS, penicillin/streptomycin and L-glutamine.

### SSTR2 expression assessment

SSTR2 transcript expression was assessed P-NEN (NT-3 and BON-1) and a non-NEN cell line (CAPAN-1) by RT/q-PCR, using HPRT as reference house keeping transcript.

### Hsa-miR-5096 Target Site Blocker transfection P-NEN cell lines

To increase SSTR2 expression in P-NEN cell lines, 3 × 105 cells were plated into 6-well dishes (NT-3 cell lines only, were plated in dishes coated with collagen type IV from Human Placenta (Sigma-Aldrich, Homefield Road, Haverhill, UK; Cat No./ID: 27663). After 24 hours, 50 and 75 nM of hsa-miR-5096 miRCURY LNA miRNA Power TSB ivr (15) - No Modification and Scramble control (Qiagen, Valencia, CA) were transfected using RNAiMAX transfection reagent (Invitrogen^{®}, Carlsbad, CA, USA) according to the manufacturer's instructions. Cells were collected 48h, 72h and 96h after transfection. The SSTR2 transcript displays 4 miR-B binding sites of two compatible sequences (SSTR2_5096_1310 and SSTR2_5096_1025) for miR-B. The TSB for each binding site (cat.n° YT0071270-FFA ; cat.n° YT0071263 respectively) was transfected either alone or in combination to shield and prevent miR-B binding to SSTR2 transcripts. TSBs in combination (50 nM) were transfected in NT-3 cell lines, cultivated with and without growth factors since the presence of growth factors significantly alters SSTR-2 expression. SSTR2 expression was also quantified at earlier times (6h,18h, and 24h) after transfection. A Fixed volume of 700 ul of Trizol^{®} reagent has been added to cell dried pellets and miRNeasy Mini Kit 50 (Qiagen, Valencia, CA Cat No./ID: 217004) was used for RNA extraction. SSTR2 expression was assessed at each time point and condition by RT/qPCR. Expression values were expressed as (Ct) values normalized to the housekeeping gene HPRT (2-ΔCT method) and then normalized to the corresponding scramble-control using the 2-ΔΔCT method. SSTR2 modulation and statistical significance were assessed by parametric t-test, comparing expression median value (+/-SD).

### Tables

**Table 1. Single miRNAs and predictors (P1, P2, P3, P) significant AUC values, sensitivity, and specificity of 18FDG/PET prediction in PAN-NEN patients. AUC: Area Under the Curve; Sens.(%); sensitivity percentage; Spec.(%): specificity percentage; C.I: Confidence interval.**

| **miRNA / predictor** | **AUC (95%CI)** | **Proposed cut-offs** | **Sens. (%)** | **Spec. (%)** |
|---|---|---|---|---|
| **hsa-miR-4311-5p** | 0.8062 (0.66-0.94) | 0.85 | 72 | 85 |
| **hsa-miR-5096** | 0.8246 (0.69-0.95) | 70 | 40 | 100 |
| **hsa-Let7i-3p** | 0.9477 (0.88-1.99) | 0.72 | 92 | 85 |
| **P1** | 0.9231 (0.83-1.00) | 0.65 | 88 | 92 |
| **P2** | 0.9508 (0.89-1.00) | 33.55 | 84 | 100 |
| **P3** | 0.8462 (0.71-0.97) | 14.9 | 88 | 77 |
| **P** | 0.9323 (0.85-1.00) | 29.6 | 80 | 100 |

**Table 2. Hsa-miR-5096 significant AUC values, sensitivity and specificity of 6-month PFS and OS in PAN-NEN patients and in the and related for ¹⁸FDG/PET positive subset predictions, treated with ¹⁷⁷Lu-DOTATE PRRT. AUC: Area Under the Curve; Sens.(%): sensitivity percentage; Spec.(%): specificity percentage; C.I: Confidence interval. 18FDG/PET (+): 18FDG/PET positive PAN-NENs subgroup.**

| **Subset** | **Clinical Endpoint** | **AUC (95%CI)** | **cut-off** | **Sens. (%)** | **Spec. (%)** |
|---|---|---|---|---|---|
| **PAN-NENs** | **PFS** | 0·8966 (0·76-1·00) | 70 | 100 | 75 |
| | **OS** | 0·8929 (0·72-1·00) | 70 | 100 | 79 |
| **¹⁸FDG/PET (+) PAN-NENs** | **PFS** | 0·8636 (0·68-1·00) | 70 | 100 | 68 |
| | **OS** | 0·8571 (0·63-1·00) | 70 | 100 | 71 |

**Table 3**

| **Clinical Endpoint** | **miR** | **AUC (95%CI)** | **cut-off method** | **Proposed cut off** | **Sens (%)** | **Spec (%)** |
|---|---|---|---|---|---|---|
| ki-67 % > 20 | hsa-miR-4311 (MIRA) | 0.8088 (0.64-0.97) | LIU/Youden | 1,44 | 100 | 71 |
| | hsa-let-7i-3p (MIRC) | 0.7868 (0.59-0.98) | LIU/Youden | 1,31 | 75 | 65 |
| | P1 | 0·8736 (0·73-1·00) | LIU/Youden | 0,98 | 100 | 59 |

**Table 4 Demographic and clinical pathological al features according to 18FDG/PET outcome: PAN-NEN case series (n= 38) p-value from Fisher exact test for categorical variables and Wilcoxon Mann-Whitney for continuous variable**

| **Variable** | | **¹⁸FDG/PET Positive (%) N=25** | **¹⁸FDG/PET Negative (%) N = 13** | **Healthy donors (%) N = 17** | **Overall** | **p-value** |
|---|---|---|---|---|---|---|
| **Age at ¹⁸PET-FDG** | | | | | | |
| | **Median (range)** | 52 (24-78) | 61 (44-79) | 42 (30-79) | 54 (30-79) | 0.046 |
| | | | | | | |

| **Gender** | | | | | | |
|---|---|---|---|---|---|---|
| | **Male** | 16 (64.0) | 11 (84.6) | 11 (64.7) | 38 (69.1) | 0.038 |
| | **Female** | 9 (36.0) | 2 (15.4) | 6 (35.3) | 17 (30.9) | |
| | | | | | | |

| **Ki67** | | | | | | |
|---|---|---|---|---|---|---|
| | **Median (range)** | 12 (1-50) | 2 (1-15) | | 8 (1-50) | 0.003 |
| | | | | | | |

| **Grading (WHO 2017)** | | | | | | |
|---|---|---|---|---|---|---|
| | **1** | 4 (16.0) | 8 (61.5) | | 12 (31.6) | 0.015 |
| | **2** | 17 (68.0) | 5 (38.5) | | 22 (57.9) | |
| | **3** | 4 (16.0) | 0 (0.0) | | 4 (10.5) | |
| | | | | | | |

| **Tumor burden** | | | | | | |
|---|---|---|---|---|---|---|
| | **Limited** | 6 (24.0) | 5 (38.5) | | 11 (28.0) | 0.680 |
| | **Moderate** | 12 (48.0) | 5 (38.5) | | 17 (44.7) | |
| | **Extensive** | 7 (28.0) | 3 (23.0) | | 10 (26.3) | |

| **Number of metastatic sites** | | | | | | |
|---|---|---|---|---|---|---|
| | **None** | 4 (16.0) | 1 (7.7) | | 5 (13.2) | 0.282 |
| | **One** | 9 (36.0) | 4 (30.8) | | 13 (34.2) | |
| | **Two** | 8 (32.0) | 8 (61.5) | | 16 (42.1) | |
| | **Three** | 4 (16.0) | 0 (0.0) | | 4 (10.5) | |
| | | | | | | |
| **Liver lesions** | | | | | | 0.817 |
| | **None** | 5 (20.8) | 2 (15.4) | | 7 (18.9) | |
| | **<6** | 7 (29.2) | 5 (38.5) | | 12 (32.4) | |
| | **>=6** | 12 (50.0) | 6 (46.1) | | 18 (48.7) | |
| | **Unknown** | 1 | 0 | | 1 | |
| | | | | | | |
| **Presence of bone metastasis** | | 6 (24.0) | 3 (23.1) | | 9 (23.7) | 1.000 |
| | | | | | | |
| **Rotterdam Index** | | | | | | |
| | **3** | 4 (16.0) | 6 (60.0) | | 10 (28.6) | 0.016 |
| | **4** | 21 (84.0) | 4 (40.0) | | 25 (71.4) | |

**Table 5 Single miRNAs and combined predictors (P1, P2, P3 and, P) expression values in P-NET ¹⁸FDG/PET positive and negative patients and Healthy Donors (HDs). Kruskall-Wallis test was used to compare the three groups. Dunn test was used for post-hoc comparisons.**

| **miR-** | **¹⁸FDG/PET Positive (%) N=25** | **¹⁸FDG/PET Negative (%) N = 13** | **Healthy donors (%) N = 17** | **Overall N=55** | **p-value** | **p-value (neg. *vs* HDs)** | **p-value (pos. *vs* HDs)** | **p-value (pos. *vs* neg)** |
|---|---|---|---|---|---|---|---|---|
| | | | | | | | | |

| **Has-miR-4311** | | | | | | | | |
|---|---|---|---|---|---|---|---|---|
| Median (range) | 1.44 (0.21-7.38) | 0.47 (0.11-1.81) | 1.13 (0.03-7.21) | 0.88 (0.03-7.38) | 0.013 | 0.086 | 0.545 | 0.005 |
| | | | | | | | | |
| | | | | | | | | |

| **Hsa-miR-5096** | | | | | | | | |
|---|---|---|---|---|---|---|---|---|
| Median (range) | 54.31 (4.98-264.79) | 22.94 (6.30-47.44) | 19.38 (1.51-323.08) | 28.76 (1.51-323.08) | 0.003 | 0.532 | 0.0312 | 0.002 |
| | | | | | | | | |
| | | | | | | | | |

| **Has-Let-7i-3p** | | | | | | | | |
|---|---|---|---|---|---|---|---|---|
| Median (range) | 1.42 (0.48-6.83) | 0.28 (0.10-1.14) | 0.67 (0.08-6.47) | 0.91 (0.08-6.83) | <0.00 1 | 0.065 | 0.046 | <0.001 |
| | | | | | | | | |
| | | | | | | | | |

| **P1** | | | | | | | | |
|---|---|---|---|---|---|---|---|---|
| Median (range) | 50.5 (2.65-736.43) | 7.89 (1.27-64.39) | 21.23 (0.12-2332.32) | 28.42 (0.12-2332.32) | 0.003 | 0.151 | 0.144 | 0.001 |
| | | | | | | | | |
| | | | | | | | | |

| **P2** | | | | | | | | |
|---|---|---|---|---|---|---|---|---|
| Median (range) | 85.16 (6.19-741.88) | 6.39 (0.67-32.05) | 15.55 (0.31-2091.76) | 35.06 (0.31-2091.76) | <0.00 1 | 0.086 | 0.024 | <0.001 |
| | | | | | | | | |
| | | | | | | | | |

| **P3** | | | | | | | | |
|---|---|---|---|---|---|---|---|---|
| Median (range) | 50.53 (2.65-736.43) | 7.89 (1.27-64.39) | 21.23 (0.12-2332.33) | 28.42 (0.12-2332.33) | 0.003 | 0.151 | 0.144 | 0.001 |
| | | | | | | | | |
| | | | | | | | | |

| **P** | | | | | | | | |
|---|---|---|---|---|---|---|---|---|
| Median (range) | 130.11 (2.69-3111.11) | 1.93 (0.13-25.06) | 18.63 (0.01-15100.2) | 34.28 (0.01-15100.2) | <0.00 1 | 0.052 | 0.097 | <0.001 |
| | | | | | | | | |

**Table 6. AUC comparison of predictors (P1, P2, P3 and P) for ¹⁸FDG/PET positivity predictions.**

| **Clinical Endpoint** | **Predictor** | **AUC (95%CI)** | **Compared predictor** | **AUC (95%CI)** | **p-value (%)** |
|---|---|---|---|---|---|
| **¹⁸FDG/PET positivity** | **P1** | 0.92 (0.83-1.00) | **P2** | 0.95 (0.89-1.00) | 0.48 |
| | | | **P3** | 0.85 (0.71-0.97) | 0.12 |
| | | | **P** | 0.93 (0.85-1.00) | 0.73 |
| | **P2** | 0.95 (0.89-1.00) | **P3** | 0.85 (0.71-0.97) | 0.02 |
| | | | **P** | 0.93 (0.85-1.00) | 0.37 |
| | **P3** | 0.84 (0.71-0.97) | **P** | 0.93 (0.85-1.00) | 0.02 |

### Results

### Patients' characteristics

To evaluate the potential role of small-non-coding molecules, such as miRNAs as prognostic biomarkers of tumor glucose metabolism and aggressiveness, 24 histologically or cytologically confirmed G1, G2 and G3 well-differentiated GEP - NET patients (WHO2017) were enrolled according to ¹⁸F-FDG/PET outcome. 12 ¹⁸F-FDG/PET positive and 12 ¹⁸F-FDG/PET negative patients were considered as "training set" for whole miRNome NGS profiling. Six out of 24 GEP-NET from the training cohort were P-NETs, while 18 out of 24 were SI-NETs. Two independent cohorts (G1, G2 and G3 well-differentiated GEP-NETs) of 30 SI-NETs and 38 P-NETs (see Table 4 for P-NET patients characteristics) were considered separately for RT/qPCR validation and downstream analysis.

### Example 1

### NGS analysis from liquid biopsy reveals circulating exosomal miRNA-signature associated with ¹⁸F-FDG/PET outcome in P-NET patients.

Whole miRNome NGS profiling was performed on 24 GEP-NETs (12 ¹⁸F-FDG/PET *positive* and 12 ¹⁸F-FDG/PET *negative).* Principal compoNETts analysis (PCA) excluded one out of 24 samples due to poor number of reads. NGS analysis identified 2588 miRNAs. Of those, 2474 miRNAs displayed at least one read in one of the samples analyzed. The bioinformatic analysis revealed hsa-miR-1246, hsa-miR-4311 and hsa-miR-485-5p as differentially expressed miRNAs (Log2FC >=1; adj. p-value < 0,1) between ¹⁸F-FDG/PET positive and negative GEP-NET patients (Figure 2A). Then, we took into consideration the contribution of the primary tumor site of origin and 17 SI-NETs and 6 P-NETs were considered separately to assess if disease specific signatures exist. Interestingly, eight miRNAs (hsa-miR-1246; hsa-miR-5096; hsa-let-7i-3p; hsa-miR-3133; hsa-miR-3614-5p; hsa-miR-483-5p; hsa-miR-519c-3p; hsa-miR-582-3p) emerged as differentially expressed between ¹⁸F-FDG/PET positive and negative P-NET patients (Figure 2B). Conversely, no miRNA correlated with ¹⁸F-FDG/PET status in the SI-NET subset. Altogether a group of 10 non redundant miRNAs were considered for further confirmatory experiments in plasma samples from 68 independent GEP-NET patients, (30 SI-NETs and 38 P-NETs) by RT/qPCR. Three circulating miRNAs (hsa-miR-4311, p < 0·001; hsa-miR-5096, p < 0·0001; hsa-let-7i-3p, p < 0·00001) significantly correlated with ¹⁸F-FDG/PET status in the P-NET subset (Figure 2C-E). In addition, in order to achieve higher prognostic power single miRNAs were combined into four predictors (P1, P2, P3 and P) as the product of fold enrichments (F.E) of each circulating miRNA. All predictors, P1 (hsa-miR-4311, hsa-let-7i-3p), P2 (hsa-mir-5096, hsa-let-7i-3p), P3 (hsa-miR-4311, hsa-mir-5096) and P (hsa-miR-4311, hsa-mir-5096, hsa-let-7i-3p) significantly correlated with ¹⁸F-FDG/PET positive status with P2 showing the highest significance (p<0·00001) (Figure 2G). In addition, hsa-miR-5096, hsa-let-7i-3p, P2 and P emerged to be significantly increased in P-NET ¹⁸F-FDG/PET positive patients as compared to healthy donors (p < 0·05) (Figure 2 D, E, G, I). Of note, the levels of hsa-let-7i-3p alone or in combination with both hsa-miR-4311 and hsa-mir-5096 in predictor P, were significantly lower in plasma of ¹⁸F-FDG/PET negative patients as compared to healthy donors (p < 0·05) (Figure 2E, I). Statistical analysis, according to ¹⁸F-FDG/PET excluded age contribution to miR-signature predictivity (Figure 11 and Table 4). Expression values for the three single miRNAs and combined predictors (P1, P2, P3 and P) in P-NET ¹⁸F-FDG/PET positive and negative patients and Healthy Donors (HDs) are reported in Table 5.

In brief, NGS whole miRNome profiling analysis and following RT/qPCR validation identified 3 miRNAs in the exosomal fraction of P-NET patients plasma associated with ¹⁸F-FDG/PET positive outcome.

### Example 2

### Identified miRNAs as companion prognostic biomarkers for ¹⁸F-FDG/PET in P-NET management (Type 0 - NIH classification)

We evaluated the predictive power of single miRNAs and predictors in relation to ¹⁸F-FDG/PET positivity. ROC analysis revealed that higher circulating expression levels of hsa-miR-4311 (AUC: 0·81; 95% CI: 0·66-0·94), hsa-miR-5096 (AUC: 0·82 95% CI: 0·69-0·95) and hsa-let-7i-3p (AUC: 0·95; 95% CI: 0·88-0·99) alone) or combined into predictors can predict ¹⁸F-FDG/PET positive outcome with AUCs between 0·81 and 0·95 (Figure 3a, b and Table 1). In particular, the combination of hsa-miR-5096 and hsa-let-7i-3p into predictor P2 (cut-off: 33·55) showed the highest predictivity (AUC: 0·95; 95% CI: 0·89-1·00) for ¹⁸F-FDG/PET positive lesions. The statistical comparison among different predictors is shown in Table 6.

### Example 3

### Identified miRNAs as independent predictors of survival for P-NET management (Type II - NIH classification)

ROC analysis was performed to evaluate the predictivity of the miRNAs signature for 6-months progression free survival (PFS) and 12-months overall survival (OS).

ROC analysis of single miRNAs and their combination into predictors (Figure 12a) showed that hsa-miR-5096 best predicts 6-month PFS (AUC: 0·90; 95% CI: 0·76 - 1·00; Figure 4a) and 12-month OS (AUC: 0·89; 95% CI: 0·72-1·00; Figure 4b) in P-NET patients treated with 177Lu-DOTATATE based PRRT . Time dependent (range: 3 - 24 month) ROC curve analysis for PFS showed that hsa-miR-5096 maintains prognostic AUC values (>= 0 7) up to 24 months (Figure 4c), while values >= 0·7 were observed for predictors P, P2 and P3 in the first 12 months only (Figure 13). Furthermore, circulating hsa-miR-5096 expression level (cut-off: 70) stratified P-NET patients with poor prognosis from responders to PRRT for PFS (p < 0·001; Figure 4D) and for OS (p < 0·05; Figure 4e). Figure 12b-l reports KM analysis of 6-mo PFS for combined predictors.

Importantly, hsa-miR-5096 in ¹⁸F-FDG/PET positive patients emerged to be an accurate predictor of PFS (AUC: 0·86; 95% CI: 0·68-1·00; Figure 5a). Specifically, an identified cut-off of 70 for hsa-miR-5096 resulted in 100% sensitivity and 68% specificity for6-mo PFS prediction in ¹⁸F-FDG/PET positive patients, identifying a subset of ¹⁸F-FDG/PET positive patients that progress even earlier and do not benefit from Lu-PRRT treatment (p< 0·01; Figure 5b). Finally, while hsa-mir-5096 represents an accurate predictor also for 12-mo OS in the ¹⁸F-FDG/PET positive subset (AUC: 0·86; 95% CI: 0·63-1·00; Figure 5b), a cut-off of 70 could not significantly stratify ¹⁸F-FDG/PET positive patients for 12-mo OS predictions (p:0·22; Figure 5d). Significant AUC values, sensitivity and specificity for 6-month PFS and OS in all and ¹⁸F-FDG/PET positive P-NET patients are shown in Table 2.

In summary, hsa-miR-5096 appeared to be an accurate and independent predictor of PFS and of OS in P-NET patients. Moreover, we candidate hsa-mir-5096 as a clinically relevant companion biomarker which can improve ¹⁸F-FDG/PET predictive power for PRRT.

### Example 4

### Hsa-miR-5096 overexpression inversely correlated with SSTR2 expression levels

To further assess its clinical impact in P-NET management, the expression levels of the miRNAs of the signature were correlated with several clinicopathological features, including ⁶⁸Ga-DOTA-PET /CT maximum standardized uptake value (SUVₘₐₓ). Interestingly, increased expression levels of circulating hsa-miR-5096 (cut-off: 70) correlated with lower ⁶⁸Ga-DOTA-PET /CT SUVₘₐₓ (p < 0·05) in P-NET patients (Figure 7a). Diagnostic imaging of SSTR2 expression with ⁶⁸Ga-DOTA-conjugated peptides is the base for the clinical management of P-NET patients to route them to PRRT. The observed inverse correlation of hsa-miR-5096 expression with SUVₘₐₓ raised the hypothesis that a direct interference with the SSTR2 transcript may exist and result in a decreased amount of SSTR2 at the plasma membrane of tumor cells. To confirm this anti correlation, a semi-automated immune-miRNA-ISH approach coupled with a dedicated pipeline of analysis (AndTool software) was set up and applied to detect and quantify hsa-miR-5096 and SSTR2 expression simultaneously on FFPE tumor tissue slices. Nine independent P-NET FFPE tumor tissue specimens were first reviewed by an expert pathologist for SSTR2 expression level. Three were negative, four were frankly positive (100%; 3+) and two displayed SSTR2 heterogeneous expression. SSTR2 expression heterogeneity level affects ⁶⁸Ga-based prognostic imaging and the efficacy of targeted therapies. *AndTool software* analysis of 9 P-NET cases, considering 10 fields per sample, identified a total number of 154154 cells with an average value of 15186±7547 analyzed cells per sample. In particular, 54% of overall analyzed cells did not express SSTR2, while 46% displayed low/heterogeneous (23%) to high (23%) expression patterns (Figure 7b). Aggregated analysis of co-occurrence confirmed that 75% of nuclei in SSTR2 negative areas express high levels of hsa-miR-5096 in contrast with 37% within SSTR2 highly expressing areas. Importantly, areas with low/moderate SSTR2 expression, which also define P-NET patients eligible for PRRT, showed an intermediate frequency of 65% hsa-mir-5096 expressing nuclei (Figure 7c). Those results show that hsa-miR-5096 is largely expressed by P-NET tumor cells and that the inverse correlation with ⁶⁸GaSUVₘₐₓ observed in blood mirrors an inverse correlation also at the tissue level.

### Example 5

### Hsa-miR-5096-5p modulates SSTR2 expression

In order to investigate the mechanism of action of hsa-miR-5096 on SSTR2 expression we performed bioinformatic analysis with on-line predictive softwares for miRNA targets. Target mining on TargetMiner, TargetScanVert, miRDB and RefSeq revealed that the 3'-UTR of SSTR2 (NCBI Gene ID: 6752; GenBank Accession:NM_001050.03) harbors 4 binding sites for hsa-miR-5096 (miRbase Accession: MIMAT0020603; Sequence: GUUUCACCAUGUUGGUCAGGC [SEQ ID N. 1]). In particular, two different sequences (GUGAAAA; GGUGAAA) are distributed on 4 sites at the 3'-UTR of the gene (723-729; 3001-3007 and 1008-1015; 2290-2260, respectively) and are predicted to be recognized by the CACUUU and CCACUUU sequences of hsa-miR-5096. The presence of the binding sites supported a possible regulation of expression via direct RNA interference in P-NET tumor cells (Figure 8a).

In order to test this hypothesis we performed *in vitro* experiments on the insulinoma NT-3 cell line, as a newly established preclinical model of well differentiated P-NET³⁰. Importantly, the neuroendocrine phenotype and morphology as well as the proliferative rate and SSTRs expression in NT-3 cells can be modulated by growth factors (bFGF/EGF) in culture. As first, we assessed SSTR2 and hsa-miR-5096 expression level in NT-3 cells and found that they inversely correlated also in NT-3 cells (Figure 7b-c). In particular, SSTR2 expression was significantly enhanced (p< 0 005) in NT-3 cells, in a condition characterized by low proliferation rate (109 +/- 0·7 days), low ki-67 percentage (2·0 %) and absence of growth factors in the culture media³⁰. Conversely, hsa-miR-5096 resulted to be significantly downregulated (p< 0·005) in these conditions (Figure 7c), confirming its negative correlation with SSTR2 expression and in agreement with our observations on P-NET tissue. In this perspective, hsa-miR-5096 seems to be part of a complex rewiring of gene expression to promote a metabolic switch and lineage differentiation in the insulinoma NT-3 cell line. To further substantiate the putative role of hsa-miR-5096 as a post-transcriptional modulator of SSTR2 expression, functional RNA interference mediated by hsa-miR-5096 was assessed in NT-3 cells, cultivated in absence of bFGF and EGF and characterized by high SSTR2 and low hsa-miR-5096 expression. As expected, the ectopic delivery of hsa-miR-5096 via miRCURY LNA transfection resulted in a significantly enhanced expression of hsa-miR-5096 compared to not-transfected and scramble mimic transfected NT-3 cells (p<0·0001; Figure 8d). Treatment of NT3 cells with scramble control significantly affected SSTR2 expression (p<0·005; Figure 8e). However, hsa-miR-5096 mimic overexpression further decreased SSTR2 mRNA level to 33% as compared to scramble treated cells at 72 hours after treatment (p<0·005; Figure 8e). These results suggest a direct mechanism where the SSTR2 3'-UTR can be actually targeted by hsa-miR-5096 reducing the stability and the dosage of SSTR2 transcripts.

In addition, we performed experiments using target site blockers aimed to shield specific sequences targeted by hsa-miR5096 on the SSTR-2 3'-UTR. Interestingly, the treatment with a single TSB targeting only two sites was unable to stabilize SSTR-2 transcript while the combination of 2 TSBs covering all 4 sites recognized by hsa-miR-5096 allowed SSTR-2 mRNA overexpression in NT-3 cells.

We previously reported that increased levels of hsa-miR-5096, via miRCURY LNA transfection, can downmodulate SSTR2 transcripts. Follow up experiments confirmed the role of hsa-miR-5096 as a druggable modulator of SSTR2 expression. Preliminary evidence in NT-3 cells shows that a combination of hsa-miR-5096 - miRCURY LNA miRNA Power TSBs designed to target all identified hsa-miR-5096 binding sites on SSTR-2 3'-UTR at 50nM promotes a significant increase of SSTR2 transcripts at 48h post transfection (p< 0,05; figure 9a-b ). BON-1, CAPAN-1 and NT-3 (cultured with growth-factors), which display lower levels of SSTR2, did not display significant SSTR2 transcript increase. In addition, to assess the potential early activity of TSBs as an early event of transcriptional machinery. SSTR2 expression was quantified at 6h,18h, and 24h in NT-3 cultivated in standard RPMI and in RPMI supplemented with growth factors. NT-3 cultivated in standard RPMI displayed a significant increase of SSTR-2 transcripts at 16h post transfection of TSBs in combination (50nM) (p<0,05; figure 10 a-b). In contrast, NT-3 cultivated with growth factors do not display SSTR2 significant increase.

### Discussion

¹⁸F-FDG/PET is considered a useful prognostic tool to assess increased glycolytic metabolism in a variety of neuroendocrine tumors, especially in P-NETs where ¹⁸F-FDG/PET positivity is associated with tumor aggressiveness and worse prognosis 1,20,27. However, ¹⁸F-FDG/PET predictive power for P-NET patients treated with PRRT is affected by the difficulty of combining data from multiple lesions in a meaningful, quantitative and objective way and active research on artificial intelligence and deep learning analysis of imaging results is trying to fill this gap 31-33. The molecular bases of this resistance and of the heterogeneity in patients' response are not known and markers associated with both features are a clinical unmet need. In 2007, US National Cancer Institute prioritized the development of novel circulating biomarkers as a key research goal, to facilitate early diagnosis, monitoring and management of NET disease³⁴. In addition, in 2016 the multinational, multidisciplinary Delphi consensus stated that measurements based on single analytes lack sensitivity, specificity and provide minimal information about the proliferative, metabolic, and metastatic features of NETs which are critical mainstays of tumor evolution. For these reasons, the combination of in vivo spatial and functional imaging of the tumor with circulating transcripts (mRNAs and ncRNAs) should be preferred and will represent a key strategy for real-time disease monitoring and prognostication in the near future³⁵.

The primary aim of our study was to find a multianalyte liquid biomarker for GEP-NET disease that correlates with ¹⁸F-FDG/PET positive status. Indeed, the retrieval of a specific combination of circulating miRNAs above a certain threshold could better recapitulate the global metabolic features of the tumor thus improving prognosis prediction. In this framework, circulating biomarkers would constitute the best companion assay for ¹⁸F-FDG/PET imaging analysis. Whole miRNome profiling from plasma of P-NET patients identified candidate miRNAs associated with ¹⁸F-FDG/PET positive outcome, 3 of which (hsa-miR-4311; hsa-mir-5096; hsa-let-7i-3p) were confirmed by RT/qPCR. Our results suggest a potential role of the miRNAs alone and combined into predictors as prognostic biomarkers for P-NET. In particular, increased exosomal plasmatic levels of P2 predictor (hsa-mir-5096; hsa-let-7i-3p) above 33·5 best predict ¹⁸F-FDG/PET positivity (AUC: 0·95), thus representing a valid surrogate biomarker for tumor aggressive metabolism in P-NET patients. Indeed, P2 (cutoff: 33·5) reports 84% sensitivity and 100% specificity in predicting higher glucose uptake 30,31, consistent with "type 0" markers of "natural history of disease", which are defined to correlate with diagnosis, prognosis and/or clinical outcome in a direct or in-direct way with the disease (NIH classification for biomarkers).

Up to now, circulating transcript based multianalyte biomarkers (e.g., NETest) do not report direct correlation with functional imaging, such as ¹⁸F-FDG - PET/CT. In this scenario, the NETest currently represents the most accurate assay to differentiate P-NET stable disease from progressive disease, taking into consideration a complex signature of 51 circulating transcripts, with a reported AUC 0·855% ²⁷.

ROC curve analysis revealed that hsa-miR-5096 per se is an accurate biomarker for PFS and OS in P-NET patients treated with ¹⁷⁷Lu-DOTATATE based PRRT (AUC: 0·90). In this context, hsa-mir-5096 assessment constitutes a low complexity and minimally invasive assay that does not require complex algorithms of analysis and interpretation. Importantly, KM survival analysis showed that circulating hsa-miR-5096 (cut-off: 70) per se distinguishes two distinct prognostic categories, regardless of ¹⁸F-FDG- PET/CT outcome: patients that will progress earlier (hsa-miR-5096 > 70) and long-term survivors (hsa-miR-5096 < 70). In this sense, hsa-miR-5096 appeared to be an independent prognostic predictor of PFS with even higher accuracy (AUC 0.90). Importantly, hsa-miR-5096 expression (cut-off: 70) was observed to stratify ¹⁸F-FDG/PET positive patients according to prognosis. In fact, patients with circulating hsa-miR-5096 level above 70 benefited the least from 177Lu-PRRT treatment. Conversely, hsa-miR-5096 score <70 and ¹⁸F-FDG/PET positive status identifies a new category of patients which benefit the most from PRRT. Given its metrics, hsa-miR-5096 can represent both a candidate type 0 and type II biomarker, which are defined as surrogate for clinical endpoints and reflect patient health, functionality, or survival (NIH classification for biomarkers). Indeed, hsa-miR-5096 measurement in the peripheral blood can be used to assess disease progression and prognosis. Crucially, high hsa-miR-5096 levels in blood and tumor tissue associates with low SSTR2 expression in P-NET patients potentially affecting PRRT efficacy.

Tumor heterogeneity is one the main hurdles that modern oncology must deal with to overcome relapse of disease. For this reason, identifying the mechanisms that sustain tumor heterogeneity becomes crucial as revealed by novel single cell technologies, multi parametric immunohistochemistry (IHC) or similar approaches. Heterogeneity can be generated through genetic, epigenetic, and post-transcriptional mechanisms. Indeed, specific exosomal resident and circulating miRNAs might be associated with P-NET patients' response to PRRT suggesting the possibility that treatment failure and RNA interference for specific targets could be intimately linked. In particular, exosomes produced by a subset of tumor cells may condition the expression of specific targets in P-NET cells. Up to now, functional imaging with ⁶⁸Ga-DOTA-conjugated peptides is used to assess SSTRs expression in patients guiding them for eligibility to PRRT¹⁷. In the present study, we observed the negative association between hsa-miR-5096 and SSTR2 expression also at the single cell level, within areas with low to absent SSTR2 expression on an independent retrospective P-NET cohort. Our results show that hsa-mir-5096 can be expressed by tumor cells and shedded in biofluids via extracellular vesicles thereby suggesting that a paracrine modulation of SSTR2 expression may exist. Of note, our in-house developed, semi-automated novel immune-miRNA-ISH technique and related open-source pipeline of analysis (ANDtool) provide standardized, operator independent, simultaneous and quantitative measurements of proteins and miRNAs in tissue specimens. Indeed, ANDtoo/ software can turn qualitative IHC/ISH analysis into quantitative measurements, and it can be used as companion software to improve pathologist evaluation workflow of FFPE biopsies. Future studies will be performed to assess its accuracy for prognostication. In addition, our results sustain that hsa-miR-5096 may not only represent a liquid surrogate marker for ⁶⁸Ga-DOTA-PET /CT SUVₘₐₓ, but also, a key determinant of SSTR2 expression which can affect clinical response to PRRT in P-NET patients. Importantly, we found that SSTR2 3'-UTR contains multiple putative binding sites specific for hsa-miR-5096. Indeed, hsa-miR-5096 ectopic overexpression in P-NET insulinoma NT-3 cells led to significant decrease of SSTR2 transcripts suggesting direct targeting and regulation. Hsa-miR-5096 is involved in a metabolic switch and lineage differentiation in the insulinoma NT-3 cell line that can be modulated and triggered by the presence of specific growth factors. Notably, the aggressive phenotype developed by NT-3 cells treated with growth factors and characterized by increased ki-67% associated with increased hsa-miR-5096 expression and with SSTR2 downregulation, consistent with data observed in patients.

Collectively, the results on P-NET patients and NT-3 cells in vitro confirm a role of hsa-miR-5096 as: i) surrogate prognostic marker of tumor aggressiveness and metabolism (type 0, NHI classification) with high association with ¹⁸F-FDG-PET/CT status; ii) independent and accurate predictor of survival (type 0 and type II, NHI classification); iii) surrogate marker associated with low ⁶⁸Ga-DOTA-PET/CT signal and low SSTR2 expression; iv) companion diagnostics of ¹⁸F-FDG-PET/CT able to identify a subset of patients (¹⁸F-FDG-PET/CT positive / hsa-miR-5096 > 70) which will not benefit of PRRT and would be better routed to alternative therapies. As we showed, hsa-miR-5096 does not act as a passive factor but it is more an active and key player in the P-NET microenvironment and ontology. High abundance of hsa-miR-5096 at tumor sites might push tumor cells to lose neuroendocrine differentiation through different mechanisms, including SSTR2 targeting and inhibition. Our findings support the hypothesis that paracrine delivery of miRNAs released by P-NET tumors via exosomes may sustain tumor heterogeneity and the development of a refractory phenotype and/or relapse to PRRT treatment.

To our knowledge NT3 cells are the only reported and validated available cell model for low grade P-NET studies and this constitutes a limitation of the present study and a general shortcoming in the P-NET arena. However, our observations in P-NET patients are backed up from the experiments performed in this cell line enforcing NT-3 value as a P-NET preclinical model.

Our study has led to a liquid, exosomal, easy to detect and robust miRNA signature with prognostic power in predicting P-NETs metabolic phenotypes, associated with glucose consumption, tumor aggressiveness and with PFS and OS in patients treated with PRRT. Our findings warrant further investigations into the role of the identified miRNAs and candidate hsa-miR-5096 as a potential target to potentiate PRRT treatment and efficacy in P-NET patients through the restoration of SSTR2 expression levels in neoplastic cells. Indeed we believe in the future it will be possible to interfere specifically with this hsa-miR5096 activity on SSTR2 transcript through the delivery of biomolecules that will shield hsa-miR-5096 binding sites on SSTR-2 3'-UTR thereby increasing its expression and susceptibility of tumor cells to PRRT. Alternatively, small molecules or small transcripts could interfere with SSTR-2 transcript stability by interacting directly with hsa-mir-5096 in tumor cells thereby preventing its interaction with its target sequences on the 3'-UTR of target genes. In addition, specific treatments resulting in the down regulation of hsa-miR-5096 expression in tumor cells or normal tissues could mimic the same regulatory effects on SSTR-2 expression.

### References

1 Andreasi V, Partelli S, Muffatti F, Manzoni MF, Capurso G, Falconi M. Update on gastroenteropancreatic neuroendocrine tumors. Dig Liver Dis 2021; 53: 171-82.
2 Dasari A, Shen C, Halperin D, et al. Trends in the incidence, prevalence, and survival outcomes in patients with neuroendocrine tumors in the United States. JAMA Oncol 2017; 3. DOI:10.1001/jamaoncol.2017.0589.
3 Young K, Starling N, Sadanandam A. The molecular biology of pancreatic neuroendocrine tumors: Challenges and translational opportunities. Semin. Cancer Biol. 2020. DOI:10.1016/j.semcancer.2019.09.024.
4 Hofland J, Kaltsas G, De Herder WW. Advances in the diagnosis and management of well-differentiated neuroendocrine tumors. Endocr. Rev. 2020. DOI:10.1210/endrev/bnz004.
5 Howe JR, Merchant NB, Conrad C, et al. The North American Neuroendocrine Tumor Society Consensus Paper on the Surgical Management of Pancreatic Neuroendocrine Tumors. Pancreas 2020. DOI:10.1097/MPA.0000000000001454.
6 Scarpa A, Chang DK, Nones K, et al. Whole-genome landscape of pancreatic neuroendocrine tumours. Nature 2017. DOI:10.1038/nature21063.
7 Partelli S, Bartsch DK, Capdevila J, et al. ENETS Consensus Guidelines for the Standards of Care in Neuroendocrine Tumours: Surgery for Small Intestinal and Pancreatic Neuroendocrine Tumours. In: Neuroendocrinology. 2017. DOI: 10.1159/000464292.
8 Scott AT, Howe JR. Evaluation and Management of Neuroendocrine Tumors of the Pancreas. Surg. Clin. North Am. 2019. DOI:10.1016/j.suc.2019.04.014.
9 Ahmed M. Gastrointestinal neuroendocrine tumors in 2020. World J. Gastrointest. Oncol. 2020. DOI: 10.4251/wjgo.v12.i8.791.
10 Nagtegaal ID, Odze RD, Klimstra D, et al. The 2019 WHO classification of tumours of the digestive system. Histopathology 2020; 76: 182-8.
11 Inzani F, Petrone G, Rindi G. The New World Health Organization Classification for Pancreatic Neuroendocrine Neoplasia. Endocrinol Metab Clin North Am 2018; 47: 463-70.
12 Choe J, Kim KW, Kim HJ, et al. What Is New in the 2017 World Health Organization Classification and 8th American Joint Committee on Cancer Staging System for Pancreatic Neuroendocrine tumors? Korean J Radiol 2019; 20: 5-17.
13 Mafficini A, Scarpa A. Genomic landscape of pancreatic neuroendocrine tumours: The International Cancer Genome Consortium. J Endocrinol 2018; 236: R161-7.
14 Guenter R, Aweda T, Carmona Matos DM, et al. Overexpression of somatostatin receptor type 2 in neuroendocrine tumors for improved Ga68-DOTATATE imaging and treatment. Surg (United States) 2020; 167: 189-96.
15 Bodei L, Ambrosini V, Herrmann K, Modlin I. Current concepts in68Ga-DOTATATE imaging of neuroendocrine tumors: Interpretation, biodistribution, dosimetry, and molecular strategies. J Nucl Med 2017; 58: 1718-26.
16 Oberg KE, Reubi J, Kwekkeboom DJ, Krenning EP. Role of somatostatins in gastroenteropancreatic neuroendocrine tumor development and therapy. Gastroenterology 2010; 139: 742-753.e1.
17 Sundin A, Arnold R, Baudin E, et al. ENETS Consensus Guidelines for the Standards of Care in Neuroendocrine Tumors: Radiological, Nuclear Medicine and Hybrid Imaging. In: Neuroendocrinology. 2017. DOI:10.1159/000471879.
18 Kwee TC, Basu S, Saboury B, Ambrosini V, Torigian DA, Alavi A. A new dimension of FDG-PET interpretation: Assessment of tumor biology. Eur J Nucl Med Mol Imaging 2011; 38: 1158-70.
19 Binderup T, Knigge U, Loft A, Federspiel B, Kjaer A. 18F-fluorodeoxyglucose positron emission tomography predicts survival of patients with neuroendocrine tumors. Clin Cancer Res 2010; 16: 978-85.
20 Severi S, Nanni O, Bodei L, et al. Role of 18FDG PET/CT in patients treated with 177Lu-DOTATATE for advanced differentiated neuroendocrine tumours. Eur J Nucl Med Mol Imaging 2013. DOI:10.1007/s00259-013-2369-z.
21 Bodei L, Kidd MS, Singh A, et al. PRRT genomic signature in blood for prediction of 177Lu-octreotate efficacy. Eur J Nucl Med Mol Imaging 2018. DOI:10.1007/s00259-018-3967-6.
22 Baum RP, Kulkarni HR, Singh A, et al. Results and adverse events of personalized peptide receptor radionuclide therapy with 90Yttrium and 177Lutetium in 1048 patients with neuroendocrine tumors. Oncotarget 2018; 9: 16932-50.
23 Kwekkeboom DJ, Kam BL, Van Essen M, et al. Somatostatin receptor-based imaging and therapy of gastroenteropancreatic neuroendocrine tumors. Endocr Relat Cancer 2010; 17. DOI: 10.1677/ERC-09-0078.
24 Bodei L, Schöder H, Baum RP, et al. Molecular profiling of neuroendocrine tumours to predict response and toxicity to peptide receptor radionuclide therapy. Lancet Oncol 2020; 21: e431-43.
25 Bocchini M, Nicolini F, Severi S, et al. Biomarkers for Pancreatic Neuroendocrine tumors (PanNETs) Management-An Updated Review. Front. Oncol. 2020. DOI: 10.3389/fonc.2020.00831.
26 Frank R, Hargreaves R. Clinical biomarkers in drug discovery and development. Nat. Rev. Drug Discov. 2003. DOI:10.1038/nrd1130.
27 Oberg K, Califano A, Strosberg JR, et al. A meta-analysis of the accuracy of a neuroendocrine tumor mRNA genomic biomarker (NETest) in blood. Ann. Oncol. 2020. DOI:10.1016/j.annonc.2019.11.003.
28 Bodei L, Kidd M, Modlin IM, et al. Measurement of circulating transcripts and gene cluster analysis predicts and defines therapeutic efficacy of peptide receptor radionuclide therapy (PRRT) in neuroendocrine tumors. Eur J Nucl Med Mol Imaging 2016; 43. DOI: 10.1007/s00259-015-3250-z.
29 Malczewska A, Bodei L, Kidd M, Modlin IM. Blood mRNA Measurement (NETest) for Neuroendocrine Tumor Diagnosis of Image-Negative Liver Metastatic Disease. J Clin Endocrinol Metab 2018. DOI:10.1210/jc.2018-01804.
30 Benten D, Behrang Y, Unrau L, et al. Establishment of the first well-differentiated human pancreatic neuroendocrine tumor model. Mol Cancer Res 2018; 16: 496-507.
31 Gao Y, Gao H, Wang G, et al. A meta-analysis of Prognostic factor of Pancreatic neuroendocrine tumors. Sci Rep 2018; 8: 1-8.
32 Gorris M, Hoogenboom SA, Wallace MB, van Hooft JE. Artificial intelligence for the management of pancreatic diseases. Dig Endosc 2020. DOI:10.1111/den.13875.
33 Luo Y, Chen X, Chen J, et al. Preoperative Prediction of Pancreatic Neuroendocrine tumors Grading Based on Enhanced Computed Tomography Imaging: Validation of Deep Learning with a Convolutional Neural Network. Neuroendocrinology 2020; 110: 338-50.
34 Modlin IM, Moss SF, Chung DC, Jensen RT, Snyderwine E. Priorities for improving the management of gastroenteropancreatic neuroendocrine tumors. J. Natl. Cancer Inst. 2008. DOI:10.1093/jnci/djn275.
35 Oberg K, Krenning E, Sundin A, et al. A delphic consensus assessment: Imaging and biomarkers in gastroenteropancreatic neuroendocrine tumor disease management. Endocr Connect 2016; 5: 174-87.

## Claims

1. An *in vitro* method for diagnosing a gastro-entero-pancreatic neuroendocrine tumor (GEP-NET) in a subject and/or determining aggressiveness of a GEP-NET in a subject diagnosed with GEP-NET and/or predicting overall survival of a subject with a GEP-NET wherein the method comprises the step of determining the expression level of one or more miRNAs in a biological sample comprising blood previously obtained from said subject, wherein the miRNAs are selected from: hsa-miR-5096, hsa-let-7i-3p and hsa-miR-4311.

2. The method according to claim 1 wherein the expression level of the one or more miRNAs is normalized in relation to the expression level of one or more reference molecule, such as a miRNA, a RNA, a DNA or other biomolecule, in particular a molecule which does not change its amount in a subject with a GEP-NET with respect to a healthy subject, preferably said reference molecule is a miRNA, more preferably it is hsa-miR-30d.

3. The method according to claim 1 or 2 further comprising the step of determining a diagnostic or prognostic response by means of comparison between the one or more miRNAs and a reference molecule.

4. The method according to anyone of claims 1-3 wherein the gastro-entero-pancreatic neuroendocrine tumor (GEP-NET) is selected from pancreatic neuroendocrine tumor (P-NET), small-intestine neuroendocrine tumor (SI-NET), intestine neuroendocrine tumor, and stomach neuroendocrine tumor.

5. An in vitro method to diagnose the presence of a Small Intestine neuroendocrine tumor (SI-NET) in a subject comprising the step of determining the expression level of hsa-miR-5096 and/or hsa-let-7i-3p in a biological sample comprising blood previously obtained from said subject, preferably said method comprising the following steps:
a. determining the expression level of hsa-miR-5096 and/or hsa-let-7i-3p in a blood sample from the subject;
b. normalizing the expression level of each miRNA determined in step a) to the expression level of a reference miRNA, RNA, DNA or other biomolecule which does not change its amount in patients bearing a SI-NET with respect to a healthy subj ect;
c. determining the presence of a Small Intestine NET (SI-NET) when the normalized expression levels of hsa-miR-5096 or of a combination of hsa-miR-5096 and hsa-let-7i-3p are equal to or greater than a predetermined cut off value.

6. An in vitro method for diagnosing an ileal primary site of origin in a subject affected by Small Intestine NET (SI-NET) comprising the step of determining the expression level of hsa-miR-5096 and/or hsa-let-7i-3p in a biological sample comprising blood previously obtained from said subject, preferably said method comprising the following steps:
a) determining the expression level of hsa-miR-5096 and/or hsa-let-7i-3p in a blood sample from the subject;
b) normalizing the expression level of each miRNA determined in step a) to the expression level of a reference miRNA, RNA, DNA or other biomolecule which does not change its amount in patients bearing a SI-NET with respect to a healthy subj ect;
c) determining the presence of an ileal primary site of origin when the normalized expression level of each of the miRNAs or of a combination thereof is equal to or greater than a predetermined cut off value.

7. An in vitro method to determine the presence of 18F-FDG-PET positive lesions in a subject affected by a gastro-entero-pancreatic neuroendocrine tumor (GEP-NET) , preferably a pancreatic neuroendocrine tumor (P-NET), comprising the step of determining the expression level of at least one of the following miRNAs: hsa-miR-5096, hsa-let-7i-3p and hsa-miR-4311 in a biological sample comprising blood previously obtained from said subject, wherein the presence of 18F-FDG-PET positive lesions is an indication of an aggressive disease, preferably said method is to determine the presence of 18F-FDG-PET positive lesions in a subject affected by a pancreatic neuroendocrine tumor (P-NET) and it comprises the following steps:
a. determining the expression level of at least one of the following miRNAs: hsa-miR-5096, hsa-let-7i-3p and hsa-miR-4311 in a blood sample from the subject;
b. normalizing the expression level of each miRNA determined in step a) to the expression level of a reference miRNA, RNA, DNA or other biomolecule which does not change its amount in patients bearing a P-NET with respect to a healthy subject ;
c. determining the presence of at least one 18F-FDG-PET positive lesion when the normalized expression level of each of the miRNAs or a combination thereof is equal to or greater than a predetermined cut off value.

8. The method according to claim 7 wherein said reference miRNA is hsa-miR-30d and said predetermined cut off value is:
0.85 for hsa-miR-4311;
70 for hsa-miR-5096;
0.72 for hsa-miR-let7i-3p;
0.65 for the combination of hsa-miR-4311 and hsa-let-7i-3p;
33.55 for the combination of hsa-mir-5096 and hsa-let-7i-3p;
14.9 for the combination of hsa-miR-4311 and hsa-mir-5096;
29.6 for the combination of hsa-miR-4311, hsa-mir-5096 and hsa-let-7i-3p,
wherein each cut-off value can vary of +/- 10%.

9. An in vitro method to predict 6 month progression-free survival (PFS) in a subject affected by a gastro-entero-pancreatic neuroendocrine tumor (GEP-NET), preferably a pancreatic neuroendocrine tumor (P-NET), comprising the step of determining the expression level of at least one of the following miRNAs: hsa-miR-5096, hsa-let-7i-3p and hsa-miR-4311 in a biological sample comprising blood previously obtained from said subject, preferably said method is to predict 6 month progression-free survival (PFS) in a subject affected by a pancreatic neuroendocrine tumor (P-NET) and it comprises the following steps:
a. determining the expression level of at least one of the following miRNAs: hsa-miR-5096, hsa-let-7i-3p and hsa-miR-4311 in a blood sample from said subject;
b. normalizing the expression level of each miRNA determined in step a) to the expression level of a reference miRNA, RNA, DNA or other biomolecule which does not change its amount in patients bearing a P-NET with respect to a healthy subj ect;
c. predicting a PFS of less than 6 months when the normalized expression level of each of the miRNAs or of a combination thereof is equal to or greater than a predetermined cut off value.

10. The method according to claim 9 wherein said reference miRNA is hsa-miR-30d and the predetermined cut off value is:
70 for hsa-miR-5096;
123.3 for the combination of hsa-mir-5096 and hsa-let-7i-3p;
142.9 for the combination of hsa-miR-4311 and hsa-mir-5096;
108.3 for the combination of hsa-miR-4311, hsa-mir-5096 and hsa-let-7i-3p,
wherein each cut-off value can vary by +/- 10%.

11. The method according to claim 9 or 10 wherein the subject is undergoing a therapy treatment targeting somatostatin receptors, more preferably a treatment with Peptide Radionuclide Receptor Therapy (PRRT).

12. An in vitro method to predict 12 month overall survival (OS) in a subject affected by a gastro-entero-pancreatic neuroendocrine tumor (GEP-NET), preferably a pancreatic neuroendocrine tumor (P-NET), comprising the step of determining the expression level of hsa-miR-5096 in a biological sample comprising blood previously obtained from said subject, preferably the method is to predict 12 month overall survival (OS) in a subject affected by a pancreatic neuroendocrine tumor (P-NET) and it comprises the following steps:
a. determining the expression level of hsa-miR-5096 in a blood sample from said subj ect;
b. normalizing the expression level of hsa-miR-5096 to a reference miRNA, RNA, DNA or other biomolecule which does not change its amount in patients bearing a P-NET with respect to a healthy subject;
c. predicting an overall survival of less than 12 months when the normalized expression level of hsa-miR-5096 is equal to or greater than a predetermined cut off value.

13. The method according to claim 12 wherein said reference miRNA is hsa-miR-30d and the predetermined cut off value is 70+/-10%.

14. An in vitro method for predicting the presence of one or more high proliferating tumor lesions in a gastro-entero-pancreatic neuroendocrine tumor (GEP-NET) comprising the step of determining the expression level of at least one of the following miRNAs: hsa-let-7i-3p and hsa-miR-4311 in a biological sample comprising blood previously obtained from said subject, preferably the method is for predicting the presence of one or more highly proliferating P-NET lesions in a subject affected by a pancreatic neuroendocrine tumor (P-NET) and it comprises the following steps:
a. determining the expression level of at least one of the following miRNAs: hsa-let-7i-3p and hsa-miR-4311 in a blood sample from said subject;
b. normalizing the expression level of each miRNA determined in step a) to the expression level of a reference miRNA, RNA, DNA or other biomolecule which does not change its amount in patients bearing a P-NET with respect to a healthy subj ect;
c. determining the presence of a highly proliferating P-NET lesion when the normalized expression level of each of the miRNAs or of a combination thereof is equal to or greater than a predetermined cut off value.

15. The method according to claim 14 wherein said reference miRNA is hsa-miR-30d and the predetermined cut off value is:
1.44 for hsa-miR-4311;
1.31 for hsa-let-7i-3p;
0.98 for the combination of hsa-miR-4311 and hsa-let-7i-3p;
wherein each cut-off value can vary of +/- 10%.

16. An in vitro method for grading a gastro-entero-pancreatic neuroendocrine tumor (GEP-NET), in particular a pancreatic neuroendocrine tumor (P-NET), in the highest grade G3 comprising the step of determining the expression level of the combination of the following miRNAs: hsa-miR-5096, hsa-let-7i-3p and hsa-miR-4311 in a biological sample comprising blood previously obtained from a subject affected by a GEP-NET tumor, preferably the method comprising the following steps:
a. determining the expression level of the combination of the following miRNAs: hsa-miR-5096, hsa-let-7i-3p and hsa-miR-4311 in a blood sample from a subject affected by pancreatic neuroendocrine tumor;
b. normalizing the expression level of each miRNA determined in step a) to the expression level of a reference miRNA, RNA, DNA or other biomolecule which does not change its amount in patients bearing a P-NET with respect to a healthy subj ect;
c. determining the presence of a G3 P-NET when the normalized expression level of the combination of miRNAs of step a) is equal to or greater than a predetermined cut off value,
wherein preferably said reference miRNA is hsa-miR-30d and the cut-off value is 57.1 +/- 10%.

17. An in vitro method for determining tumor burden in a subject affected by a gastro-entero-pancreatic neuroendocrine tumor (GEP-NET), preferably a pancreatic neuroendocrine tumor (P-NET), comprising the step of determining the expression level of at least one of the following miRNAs: hsa-let-7i-3p and hsa-miR-4311 in a biological sample comprising blood obtained from said subject, preferably the method comprising the following steps:
a. determining the expression level of at least one of the following miRNAs: hsa-let-7i-3p and hsa-miR-4311 in a blood sample from said subject;
b. normalizing the expression level of each miRNA determined in step a) to the expression level of a reference miRNA, RNA, DNA or other biomolecule which does not change its amount in patients bearing a P-NET with respect to a healthy subj ect;
c. determining the presence of a high tumor burden when the normalized expression level of the combination of miRNAs of step a) is equal to or greater than a predetermined cut off value.

18. An in vitro method to predict response to therapy in a subject affected by a gastro-entero-pancreatic neuroendocrine tumor (GEP-NET), in particular a pancreatic neuroendocrine tumor (P-NET), comprising the step of determining the expression level of hsa-miR-5096, in a biological sample comprising blood previously obtained from said subject, preferably the method comprising the following step:
a) determining the expression level of hsa-miR-5096 in a blood sample from said subj ect;
b) normalizing the expression level of the miRNA determined in step a) to the expression level of a reference miRNA, RNA, DNA or other biomolecule which does not change its amount in patients bearing a P-NET with respect to a healthy subject ;
c) predicting a positive response to Peptide Radionuclide Receptor Therapy (PRRT) when the normalized expression level of hsa-miR-5096 is lower than a predetermined cut off value,
wherein preferably said reference miRNA is hsa-miR-30d and the cut off value is 70 +/-10%.

19. Use of hsa-miR-5096, hsa-let-7i-3p and/or hsa-miR-4311 as biomarkers for diagnosing a gastro-entero-pancreatic neuroendocrine tumor (GEP-NET) in a subject and/or for determining aggressiveness of a GEP-NET in a subject diagnosed with GEP-NET and/or for predicting overall survival of a subject with a GEP-NET and/or for predicting response to therapy in a subject with a GEP-NET, preferably as biomarkers for:
▪ determining the presence in a subject of a gastro-entero-pancreatic neuroendocrine tumor (GEP-NET), in particular of a pancreatic neuroendocrine tumor (P-NET) or of a Small Intestine neuroendocrine tumor (SINET);
▪ diagnosing an ileal primary site of origin in a subject affected by Small Intestine NET (SINET) ;
▪ determining the presence of 18F-FDG-PET positive lesions in a subject affected by a pancreatic neuroendocrine tumor (P-NET);
▪ predicting 6 month progression-free survival (PFS) in a subject affected by a pancreatic neuroendocrine tumor (P-NET) who is undergoing a treatment with Peptide Radionuclide Receptor Therapy (PRRT);
▪ predicting 12 month overall survival (OS) in a subject affected by a pancreatic neuroendocrine tumor (P-NET) who is undergoing a treatment with Peptide Radionuclide Receptor Therapy (PRRT);
▪ predicting 6 month progression-free survival (PFS) in a subject affected by a pancreatic neuroendocrine tumor (P-NET) who is undergoing a treatment with Peptide Radionuclide Receptor Therapy (PRRT) and is positive to ¹⁸F-FDG-PET;
▪ predicting the presence of highly proliferating P-NET lesions in a subject affected by a pancreatic neuroendocrine tumor (P-NET);
▪ grading a pancreatic neuroendocrine tumor (P-NET);
▪ predicting tumor burden in a subject affected by a pancreatic neuroendocrine tumor (P-NET); and/or
▪ predicting response to Peptide Radionuclide Receptor Therapy (PRRT) in a subject affected by a pancreatic neuroendocrine tumor (P-NET).

20. An inhibitor of hsa-miR-5096 for use for the treatment of a cancer, preferably a gastro-entero-pancreatic neuroendocrine tumor (GEP-NET), more preferably a pancreatic neuroendocrine tumor (P-NET).

21. The inhibitor for the use according to claim 20 wherein said inhibitor is an agent capable of binding and blocking one or more of the binding sites of hsa-miR-5096 on the SSTR-2 transcript, preferably it is a LNA Target Site Blocker (TSB).

22. A kit or a device for carrying out any of the methods of claims 1-18, preferably said kit containing one or more of the followings:
- agents and/or tools to obtain plasma exosome-enriched fraction from a blood sample previously isolated from a subject;
- agents and/or tools to isolate target miRNAs;
- agents and/or tools for the dosage of each miRNA, for example primers and probes specific for the target miRNAs and/or useful for RT-PCR.
